# EUROPÄISCHE PATENTSCHRIFT

(11) **EP 1 381 639 B1**
(45) Veröffentlichungstag und Bekanntmachung des Hinweises auf die Patenterteilung: **08.09.2004**
(21) Anmeldenummer: 02753715.8
(22) Anmeldetag: 22.03.2002
(51) Int. Cl.: C08G 61/00, C09K 11/06, H05B 33/14, H01L 51/30

(54) **KONJUGIERTE POLYMERE ENTHALTEND SPIROBIFLUOREN-EINHEITEN UND FLUOREN-EINHEITEN UND DEREN VERWENDUNG**
CONJUGATED POLYMERS CONTAINING SPIROBIFLUORENE UNITS AND FLUORENE UNITS, AND THE USE THEREOF
POLYMERES CONJUGUES CONTENANT DES UNITES SPIROBIFLUORENE ET DES UNITES FLUORENE ET LEUR UTILISATION

(30) Priorität: 24.03.2001 DE 10114477
(43) Veröffentlichungstag der Anmeldung: 21.01.2004
(73) Patentinhaber: Covion Organic Semiconductors GmbH, 65926 Frankfurt (DE)
(72) Erfinder: TREACHER, Kevin, 65779 Kelkheim/Münster (DE); BECKER, Heinrich, 61479 Glashütten (DE); STÖSSEL, Philipp, 65929 Frankfurt (DE); SPREITZER, Hubert, 68519 Viernheim (DE); FALCOU, Aurelie, 55128 Mainz (DE); PARHAM, Amir, 60436 Frankfurt (DE); BÜSING, Arne, 60489 Frankfurt (DE)
(74) Vertreter: Luderschmidt, Schüler & Partner GbR
(86) Internationale Anmeldenummer: PCT/EP2002/003221
(87) Internationale Veröffentlichungsnummer: WO 2002/077060

(56) Entgegenhaltungen:
- EP-A- 0 676 461
- EP-A- 0 707 020
- EP-A- 1 138 746
- WO-A-00/46321
- HEEGER ET AL.: "Spiro-functionalized Polyfluorene Derivatives as Blue-Light-Emitting materials" ADVANCED MATERIALS, Bd. 12, Nr. 11, 22. Mai 2000 (2000-05-22), Seiten 828-831, XP002209789

## Beschreibung

Seit ca. 10 Jahren läuft eine breitangelegte Forschung zur Kommerzialisierung von Anzeige- und Beleuchtungselementen auf Basis Polymerer (Organischer) Leuchtdioden (PLEDs). Ausgelöst wurde diese Entwicklung durch die Grundlagenentwicklungen, welche in EP 423 283 (WO 90/13148) offenbart sind. Im Gegensatz zu den niedermolekularen Organischen Leuchtdioden (OLEDs), bei denen die Markteinführung bereits erfolgt ist, wie die im Markt erhältlichen AutoRadios mit "Organischem Display" der Firma Pioneer belegen, steht diese bei den PLEDs noch bevor. Es sind hier immer noch deutliche Verbesserungen nötig, um diese Displays zu einer echten Konkurrenz zu den derzeit marktbeherrschenden Flüssigkristallanzeigen (LCD) zu machen bzw. diese zu überflügeln.

In EP-A-0 423 283, EP-A-0 443 861, WO 98127136, EP-A-1 025 183 und WO 99124526 werden als konjugierte polymere Emitter Poly-Arylen-Vinylen-Derivate offenbart.
In EP-A-0 842 208, WO 99154385, WO 00122027, WO 00/22026 und WO 00/46321 werden als konjugierte polymere Emitter Poly-Fluoren-Derivate offenbart.
In EP-A-0 707 020 und EP-A-0 894 107 werden als konjugierte polymere Emitter Poly-Spirobifluoren-Derivate offenbart.

Konjugierte Polymere im Sinne dieser Erfindung sollen Polymere sein, die in der Hauptkette hauptsächlich sp²-hybridisierte Kohlenstoffatome, die auch durch entsprechende Heteroatome ersetzt sein können, enthalten. Dies ist gleichbedeutend mit dem abwechselnden Vorliegen von Doppel- und Einfachbindungen in der Hauptkette. Hauptsächlich meint, daß natürlich auftretende Defekte, die zu Konjugationsunterbrechungen führen, den Begriff "Konjugierte Polymere" nicht entwerten. Es sind jedoch keine Polymere, welche absichtlich eingefügte größere Mengen an nichtkonjugierten Segmenten enthalten, gemeint. Des weiteren wird in diesem Anmeldetext ebenfalls als konjugiert bezeichnet, wenn sich in der Hauptkette z. B. Arylamineinheiten und/oder bestimmte Heterocyclen (d. h. Konjugation über N-, O-, oder S-Atome) und/oder Metallorganische Komplexe (d. h. Konjugation über das Metallatom) befinden. Hingegen würden Einheiten wie einfache (Thio)Etherbrücken, Esterverknüpfungen, Amid- oder Imidverknüpfungen eindeutig als nicht-konjugierte Segmente definiert.

Der allgemeine Aufbau von PLEDs ist in den o. g. Anmeldeschriften bzw. Patenten wiedergegeben und auch weiter unten noch näher erläutert. Weitere Verfeinerungen (beispielsweise Passiv-Matrix-Adressierung, Aktiv-Matrix-Adressierung) sind ebenfalls bereits bekannt, sind aber für die weitere Beschreibung der hier vorliegenden Anmeldung nicht entscheidend.
Derzeit wird die Kommerzialisierung von sowohl einfarbigen als auch vollfarbigen Displays basierend auf PLEDs erwogen. Während die einfarbigen Displays eventuell durch einfache Beschichtungstechnologien (wie z. B. Rackeln, Spin-Coaten) erzeugt werden können, ist bei mehr- bzw. vollfarbigen Anzeigeelementen der Einsatz von Druckverfahren (z. B. Tintenstrahldrucken, Off-Set-Drucken, Tiefdruckverfahren) sehr wahrscheinlich. All diese Verfahren benötigen jedoch lösliche Polymere.

Die konjugierten Polymere gemäß den o. g. Anmeldungen zeigen zum Teil schon gute Eigenschaften für die aufgeführten Anwendungen auf.
Wichtige Eigenschaften sind hierbei v. a. folgende:
- Hohe Leucht- und Energieeffizienz in der Verwendung in PLEDs.
- Lange Operative Lebensdauer in der Verwendung in PLEDs.
- Niedrige Betriebsspannung.
- Gute Lagerstabilität, sowohl in der Verwendung in PLEDs, als auch vor deren Einbringen in entsprechende Vorrichtungen.
- Gute Löslichkeit in organischen Lösemitteln um überhaupt ein entsprechendes Beschichtungsverfahren zu ermöglichen.
- Vernünftige Zugänglichkeit um die wirtschaftliche Verwendung in Massenprodukten zu ermöglichen.
- Erzielbarkeit verschiedener Farben, um vollfarbige Anzeigeelemente (Displays) zu ermöglichen.

Es wurde nun überraschend gefunden, daß eine neue Klasse von konjugierten Polymeren sehr gute und häufig den o. g. Stand der Technik übertreffende Eigenschaften aufweist. Diese Polymere und deren Verwendung in PLEDs sind Gegenstand der vorliegenden Erfindung.

Gegenstand der Erfindung sind konjugierte Polymere, die neben Einheiten der Formel (I) 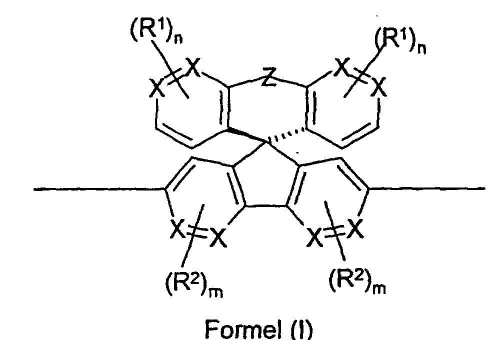 auch solche der Formel (II) enthalten, 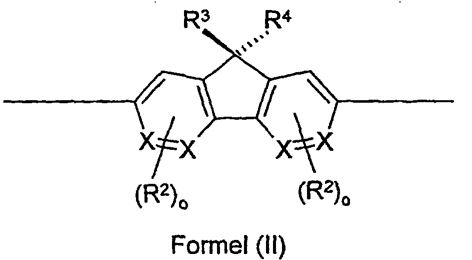 mit der Maßgabe, daß der Anteil der Wiederholeinheiten vom Typ Formel (I) und Formel (II) zusammen mindestens 20%, bevorzugt mindestens 30%, besonders bevorzugt mindestens 50% aller Wiederholeinheiten im Polymer ausmachen, und daß dabei das Verhältnis der Wiederholeinheiten vom Typ Formel (I) zum Typ Formel (II) im Bereich von 1:10 bis 10:1, bevorzugt 1:5 bis 5:1, besonders bevorzugt 1:2 bis 2:1 liegt,
und die Symbole und Indizes die folgende Bedeutung haben:
- X: ist bei jedem Auftreten gleich oder verschieden CH, CR¹ oder N,
- Z: ist bei jedem Auftreten gleich oder verschieden einer chemischen Einfachbindung, einer CR³R⁴-Gruppierung, einer -CR³R⁴-CR³R⁴-Gruppierung, einer -CR³=CR⁴-Gruppierung, O, S, N-R⁵, C=O, C=CR³R⁴ oder SiR³R⁴;
- R¹: ist bei jedem Auftreten.gleich oder verschieden eine geradkettige, verzweigte oder cyclische Alkyl- oder Alkoxykette mit 1 bis 22 C-Atomen, in der auch ein oder mehrere nicht benachbarte C-Atome durch N-R⁵, O, S, -CO-O-, O-CO-O ersetzt sein können, wobei auch ein oder mehrere H-Atome durch Fluor ersetzt sein können, eine Aryl- oder Aryloxygruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können, oder Cl, F, CN, N(R⁵)₂, wobei auch zwei oder mehrere Reste R¹ miteinander ein Ringsystem bilden können;
- R²: ist bei jedem Auftreten gleich oder verschieden eine geradkettige, verzweigte oder cyclische Alkyl- oder Alkoxykette mit 1 bis 22 C-Atomen, in der auch ein oder mehrere nicht benachbarte C-Atome durch N-R⁵, O, S, -CO-O-, O-CO-O ersetzt sein können, wobei auch ein oder mehrere H-Atome durch Fluor ersetzt sein können, eine Aryl- oder Aryloxygruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können, oder CN;
- R³, R⁴: sind bei jedem Auftreten gleich oder verschieden H, eine geradkettige, verzweigte oder cyclische Alkylkette mit 1 bis 22 C-Atomen, in der auch ein oder mehrere nicht benachbarte C-Atome durch N-R⁵, O, S, -CO-O-, O-CO-O ersetzt sein können, wobei auch ein oder mehrere H-Atome durch Fluor ersetzt sein können, eine Arylgruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können, oder CN; die beiden Reste R³ und R⁴ können zusammen auch einen Ring ausbilden, der allerdings nicht zu Strukturen gemäß Formel (I) führt;
- R⁵: ist bei jedem Auftreten gleich oder verschieden H, eine geradkettige, verzweigte oder cyclische Alkylkette mit 1 bis 22 C-Atomen, in der auch ein oder mehrere nicht benachbarte C-Atome durch O, S, -CO-O-, O-CO-O ersetzt sein können, wobei auch ein oder mehrere H-Atome durch Fluor ersetzt sein können, eine Arylgruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können;
- n: ist bei jedem Auftreten gleich oder verschieden 0,1, 2, 3, oder 4, bevorzugt 0, 1, oder 2, besonders bevorzugt 1 oder 2;
- m: ist bei jedem Auftreten gleich oder verschieden 0, 1, 2, oder 3, bevorzugt 0, 1, oder 2, besonders bevorzugt 0 oder 1;
- o: ist bei jedem Auftreten gleich oder verschieden 0, 1, 2, oder 3, bevorzugt 0, 1, oder 2, besonders bevorzugt 0 oder 1,
und der Maßgabe, daß bei mindestens einer Einheit gemäß Formel (I) mindestens ein Index n und/oder m von 0 verschieden ist.

Auch wenn dies aus der Beschreibung hervorgeht, sei hier noch mal explizit darauf verwiesen, daß sowohl die Struktureinheiten gemäß Formel (I), als auch jene gemäß Formel (II) unsymmetrisch substituiert sein können, d. h. an einer Einheit unterschiedliche Substituenten R¹ und/oder R² vorhanden sein können, bzw. diese auch unterschiedliche Stellungen auf den jeweils beiden Seiten haben können.

Die Synthese der entsprechenden Monomeren ist z. B. in den oben bereits genannten Anmeldeschriften und Patenten ausführlich beschrieben.
So können beispielsweise Monomere, die dann im Polymer Strukturen gemäß Formel (I) ergeben, gemäß EP 676.461, EP 707.020, EP 894.107 und den darin zitierten Literaturstellen synthetisiert werden.
Des weiteren können beispielsweise Monomere, die dann im Polymer Strukturen gemäß Formel (II) ergeben, gemäß EP-A-0 842 208, WO 99/54385, WO 00/22026 und den darin zitierten Literaturstellen synthetisiert werden.

Die erfindungsgemäßen Polymere weisen v. a. gegenüber den o. g. Poly-Spirobifluorenen (welche zwar Einheiten gemäß Formel (I), nicht aber solche gemäß Formel (II) enthalten) und Polyfluorenen (welche nur Einheiten gemäß Formel (II), nicht aber solche gemäß Formel (I) enthalten) folgende Vorteile auf:
(1) Es wurde überraschend gefunden, daß die erfindungsgemäßen Polymere (bei ansonsten gleicher oder ähnlicher Struktur) höhere Leuchteffizienzen in der Anwendung aufweisen (vgl. z. B. die Daten für das erfindungsgemäße Polymer P3 mit denjenigen der Vergleichspolymere V1 und V6; analoger Vergleich für P8 mit V2; siehe Beispiele, Tabelle in Teil B). Dies ist von enormer Bedeutung, da somit entweder gleiche Helligkeit bei geringerem Energieverbrauch erzielt werden kann, was vor allem bei mobilen Applikationen (Displays für Handys, Pager, PDA etc.) sehr wichtig ist. Umgekehrt erhält man bei gleichem Energieverbrauch höhere Helligkeiten, was beispielsweise für Beleuchtungsanwendungen interessant sein kann.
(2) Des weiteren hat sich auch überraschend gezeigt, daß wiederum im direkten Vergleich die erfindungsgemäßen Polymere höhere operative Lebensdauern aufweisen (vgl. w. o. die Daten für das erfindungsgemäße Polymer P3 mit denjenigen der Vergleichspolymere V1 und V6; analoger Vergleich für P8 mit V2; siehe Beispiele, Tabelle in Teil B).
(3) Auch vom Löslichkeitsverhalten (z. B. Gelierungstemperatur bei gegebener Konzentration, Viskosität bei gegebener Konzentration) sind die erfindungsgemäßen Polymere den bekannten Polymeren gleichwertig bzw. überlegen (vgl. z. B. die Daten für das erfindungsgemäße Polymer P8 mit denjenigen des Vergleichspolymers V2; analoger Vergleich für P1 bzw. P3 mit V1 und V6; analoger Vergleich für P6 mit V5; siehe Beispiele, Tabelle in Teil B).
(4) Die Zugänglichkeit und die Erzielbarkeit von Farben ist bei den erfindungsgemäßen Polymeren gleichwertig zum Stand der Technik. Dies stellt somit zwar keinen Vorteil dar, die genannten Vorteile unter (1) bis (3) zeitigen aber auch keine negativen Auswirkung, was ja häufig bei technischen Optimierungen eintritt.

Die erfindungsgemäßen Polymere weisen in der Regel 10 bis 10000, bevorzugt 50 bis 5000, besonders bevorzugt 50 bis 2000 Wiederholeinheiten auf.

Die nötige Löslichkeit wird v. a. durch die Substituenten R¹, R³ und/oder R⁴ gewährleistet. Falls Substituenten R² vorhanden sind, tragen auch diese zur Löslichkeit bei.
Um ausreichende Löslichkeit zu gewährleisten ist es nötig, daß im Durchschnitt pro Wiederholeinheit mindestens 2 nicht-aromatische C-Atome in den Substituenten vorhanden sind. Bevorzugt sind dabei mindestens 4, besonders bevorzugt mindestens 8 C-Atome. Einzelne dieser C-Atome können auch noch durch O oder S ersetzt sein. Dies kann aber durchaus bedeuten, daß ein gewisser Anteil von Wiederholeinheiten, sowohl solche gemäß Formel (I) oder (II) als auch anderer Strukturtypen, keine weiteren nicht-aromatischen Substituenten trragen.
Um die Morphologie des Filmes nicht zu verschlechtem ist es bevorzugt, keine langkettigen Substituenten mit mehr als 12 C-Atomen in einer linearen Kette zu haben, bevorzugt keine mit mehr als 8 C-Atome, besonders bevorzugt keine mit mehr als 6 C-Atome.
Nicht-aromatische C-Atome sind, wie in der Beschreibung für bspw. R¹, in entsprechenden geradkettigen, verzweigten oder cyclischen Alkyl- oder Alkoxyketten enthalten.

Bevorzugt sind erfindungsgemäße Polymere bei denen für das Symbol X = C-H oder C-R¹ gilt.
Des weiteren bevorzugt sind erfindungsgemäße Polymere bei denen das Symbol Z für eine chemische Einfachbindung steht.

Bevorzugt sind weiterhin erfindungsgemäße Polymere, bei denen gilt:
- R¹: ist bei jedem Auftreten gleich oder verschieden eine geradkettige, verzweigte oder cyclische Alkyl- oder Alkoxykette mit 1 bis 10 C-Atomen, wobei auch ein oder mehrere H-Atome durch Fluor ersetzt sein können, eine Arylgruppe mit 6 bis 14 C-Atomen, welche auch durch ein oder mehrere nicht-aromatische Reste R¹ substituiert ist.

Besonders bevorzugt sind weiterhin erfindungsgemäße Polymere, bei denen gilt:
- R¹: ist bei jedem Auftreten gleich oder verschieden eine geradkettige oder verzweigte Alkyl- oder Alkoxykette mit 1 bis 8 C-Atomen, oder eine Arylgruppe mit 6 bis 10 C-Atomen, welche auch durch ein oder mehrere nicht-aromatische Reste R¹ substituiert ist;
- n: ist gleich oder verschieden 1 oder 2.

Bevorzugt sind weiterhin erfindungsgemäße Polymere, bei denen gilt:
- R²: ist bei jedem Auftreten gleich oder verschieden eine geradkettige oder verzweigte Alkyl- oder Alkoxykette mit 1 bis 10 C-Atomen, wobei auch ein oder mehrere H-Atome durch Fluor ersetzt sein können, eine Aryl- oder Aryloxygruppe mit 6 bis 14 C-Atomen, welche auch durch ein oder mehrere nicht-aromatische Reste R¹ substituiert sein können, oder CN;
- o, m: ist bei jedem Auftreten gleich oder verschieden 0 oder 1.

Besonders bevorzugt sind weiterhin erfindungsgemäße Polymere, bei denen gift:
- R²: ist bei jedem Auftreten gleich oder verschieden eine geradkettige oder verzweigte Alkyl- oder Alkoxykette mit 1 bis 8 C-Atomen, wobei auch ein oder mehrere H-Atome durch Fluor ersetzt sein können, eine Arylgruppe mit 6 bis 10 C-Atomen, welche auch durch ein oder mehrere nicht-aromatische Reste R¹ substituiert sein können;
- o, m: ist bei jedem Auftreten gleich oder verschieden 0 oder 1, wobei für mindestens 50%, bevorzugt für mindestens 70%, ganz besonders bevorzugt für mindestens 90% aller im Polymer vorhandenen Wiederholeinheiten gemäß Formel (1) und (II) gilt, daß o und m gleich 0 ist.

Des weiteren bevorzugt sind erfindungsgemäße Polymere, bei denen gilt:
- R³, R⁴: sind bei jedem Auftreten gleich oder verschieden eine geradkettige, verzweigte oder cyclische Alkylkette mit 1 bis 10 C-Atomen, in der auch ein oder mehrere nicht benachbarte C-Atome durch O ersetzt sein können, wobei auch ein oder mehrere H-Atome durch Fluor ersetzt sein können, eine Arylgruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können.

Besonders bevorzugt sind hierbei erfindungsgemäße Polymere, bei denen gilt:
- R³, R⁴: sind bei jedem Auftreten gleich oder verschieden eine Arylgruppe mit 6 bis 14 C-Atomen, welche auch durch ein oder mehrere nicht-aromatische Reste R¹ substituiert sein können.

Ganz besonders bevorzugt sind hierbei erfindungsgemäße Polymere, bei denen gilt:
- R³, R⁴: sind bei jedem Auftreten gleich oder verschieden eine Arylgruppe mit 6 bis 14 C-Atomen, welche auch durch ein oder mehrere nicht-aromatische Reste R¹ substituiert sein können und bei denen jeweils an einer Einheit gemäß Formel (II) die Substituenten R³ und R⁴ voneinander unterschiedlich sind. Unterschiedlich sind hierbei auch schon Substituenten, die zwar gleiche Arylgruppen aufweisen, aber unterschiedliche Reste R¹ tragen, bzw. diese in unterschiedlichen Stellung tragen.

Gerade diese letzte Bevorzugung führt zu einer enormen Verbesserung der Lösungseigenschaften, ohne gleichzeitig morphologische Eigenschaften zu verschlechtern .

Die erfindungsgemäßen Polymere per se sind bereits Copolymere, die mindestens zwei verschiedene Wiederholeinheiten (Formel (1) und Formel (II)) besitzen. Darüber hinaus sind auch Copolymere bevorzugt, die noch weitere, andere Wiederholeinheiten besitzen, die andere Strukturen als diejenigen gemäß Formel (1) oder (II) aufweisen. Derartige weitere Strukturen werden unten noch näher erläutert. Die erfindungsgemäßen Copolymeren können nun statistische, alternierende oder auch blockartige Strukturen aufweisen, oder auch mehrere dieser Strukturen abwechselnd besitzen.
Es sind aber auch erfindungsgemäße Copolymere bevorzugt, die ein oder mehrere verschiedene Strukturen gemäß Formel (I) und ein oder mehrere verschiedene Strukturen gemäß Formel (II) aufweisen.
Durch das Verwenden mehrerer verschiedener Strukturelemente können Eigenschaften wie Löslichkeit, Festphasenmorphologie, Farbe etc. eingestellt werden.

Bevorzugte Copolymere, die noch weitere Strukturelemente neben denen gemäß Formel (I) und Formel (II) enthalten, sind solche, bei denen mindestens ein weiteres Strukturelement Ladungstransporteigenschaften aufweist.
Im Sinne dieses Anmeldetextes soll unter solchen Strukturelementen folgendes verstanden werden: würde man aus diesen Strukturelementen HOMOPOLYMERE oder -OLIGOMERE erzeugen, hätten diese - zumindest für einen Ladungsträger, d. h. entweder Elektronen oder Löcher - eine höhere Ladungsträgermobilität, wie dies bei einem erfindungsgemäßen Polymer, welches ausschließlich aus Strukturelementen gemäß Formeln (I) und Formeln (II) besteht, der Fall ist.
Bevorzugt ist die Ladungsträgerbeweglichkeit (gemessen in cm²/(V*s)) mindestens einen Faktor 10, besonders bevorzugt mindestens einen Faktor 50 größer.

Strukturelemente, die Lochtransporteigenschaften aufweisen, sind beispielsweise Triarylaminderivate, Benzidinderivate, Tetraarylen-para-phenylendiaminderivate, Phenothiazinderivate, Phenoxazinderivate, Dihydrophenazinderivate, Thianthrenderivate, Benzo-p-dioxinderivate, Phenoxathiinderivate, Carbazolderivate, Azulenderivate, Thiophenderivate, Pyrrolderivate, Furanderivate und weitere O, S oder N-haltige Heterocyclen, mit hochliegenden HOMO (HOMO = höchstliegendes besetztes Molekülorbital); bevorzugt haben diese Heterocyclen ein HOMO bei weniger als 6.0 eV (gegen Vakuumlevel), besonders bevorzugt von weniger als 5.5 eV.

Bevorzugt sind dabei erfindungsgemäße Polymere, die noch mindestens eine Struktureinheit gemäß den Formeln (111) bis (XIX) enthalten. Der Anteil dieser Strukturelemente ist dabei mindestens 1%, bevorzugt mindestens 5%. Der maximale Anteil ist dabei höchstens 70%, bevorzugt höchstens 50%. Auch diese Struktureinheiten können im Polymer statistisch, alternierend oder blockartig eingebaut sein. 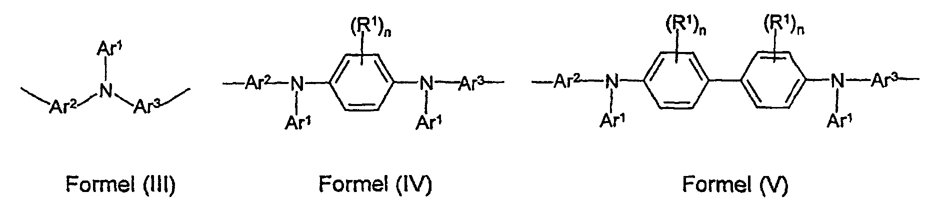 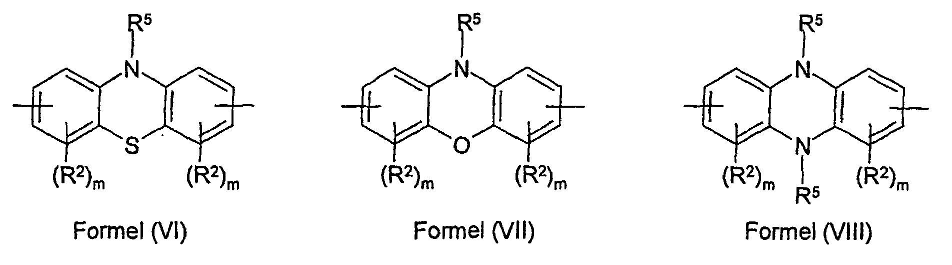 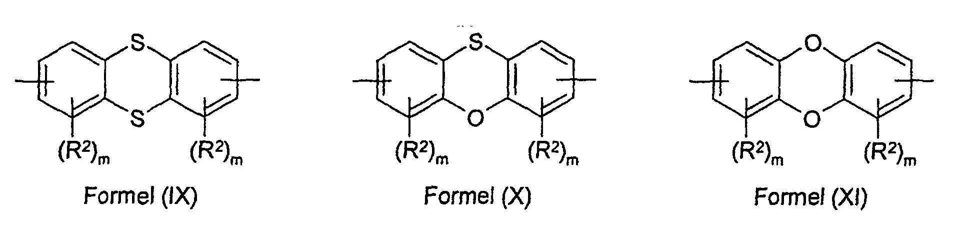 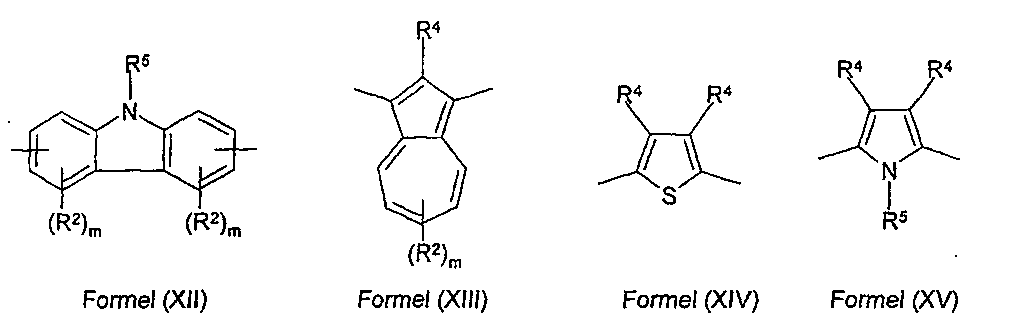 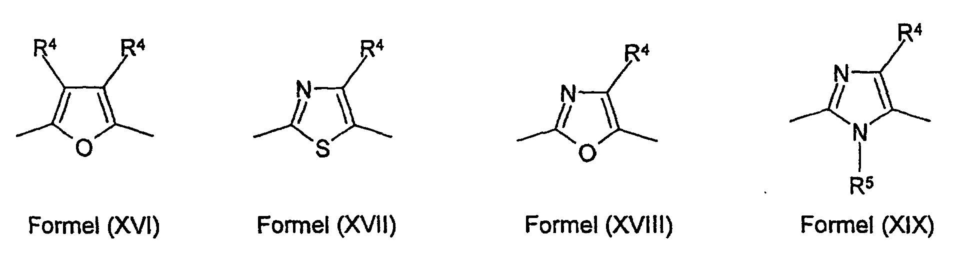 wobei die Symbole R¹ bis R⁵ und die Indizes n und m die unter Formel (1) und Formel (II) genannten Bedeutungen haben, und die weiteren Symbole
- Ar¹, Ar², Ar³: bei jedem Auftreten gleich oder verschieden aromatischen oder heteroaromatischen Kohlenwasserstoffen mit 2 bis 40 C-Atomen, welche auch mit einem oder mehreren nicht-aromatischen Resten R¹ substituiert sein können, sind.
- Ar¹, Ar², Ar³: sind dabei bevorzugt substituierte oder unsubstituierte aromatische Kohlenwasserstoffe, welche 6 bis 20 C-Atome aufweisen, besonders bevorzugt entsprechende Benzol-, Naphthalin-, Anthracen-, Pyren- oder Perylenderivate.

Die Art des Einbaus dieser Strukturen ist bei etlichen schon direkt vorgegeben (siehe z. B. Formeln (III) bis (V) und Formeln (XIII) bis (XIX)). Bei anderen Strukturen sind jeweils mehrere Möglichkeiten erfindungsgemäß. Allerdings gibt es bei diesen auch bevorzugte Einbauweisen:
So ist bei den N-haltigen tricyclischen Heterocyclen (Formeln (VI) bis Formel (VIII)) jeweils die Verknüpfung via C-Atomen in para-Stellung zum Stickstoff (d. h. bei Phenothiazin- und Phenoxazinderivaten: 3,7-Position; bei
Dihydrophenazinderivaten: 2,7- bzw. 3,7-Positon) bevorzugt. Analoges gilt auch bei Carbazolderivaten (Formel (XII)). Für die O- und/oder S-haltigen Tricyclen (Formeln (IX) bis (XI)) sind hingegen sowohl ortho- bzw. para-Positionen zu einem der Heteroatome bevorzugt.

Monomere für den Einbau von Struktureinheiten gemäß Formel (III), Formel (IV) und Formel (V) können beispielsweise gemäß WO98/06773 synthetisiert werden.
Monomere für den Einbau von Struktureinheiten gemäß Formel (VI), Formel (VII) und Formel (VIII) können beispielsweise gemäß M. Jovanovic et al., *J. Org. Chem.* **1984,** *49*, 1905 und H. J. Shine et al., *J. Org.* Chem. **1979**, *44*, 3310 synthetisiert werden.
Monomere für den Einbau von Struktureinheiten gemäß Formel (IX) und Formel (X) können beispielsweise gemäß J. Lovell et al., *Tetrahedron* **1996,** *52*, 4745, US-A- 4.505.841 und den darin genannten Literaturstellen synthetisiert werden.
Monomere für den Einbau von Struktureinheiten gemäß Formel (XI) können beispielsweise gemäß A. D. Kuntsevich et al., *Zh. Obshch. Khim.* **1994,** *64*, 1722 und A. D. Kuntsevich et al., *Dokl. Akad. Nauk* **1993,** *332*, 461 synthetisiert werden. Halogenierte Monomere für den Einbau von Struktureinheiten gemäß Formel (XII) sind in großer Breite in der Literatur bekannt und teilweise sogar kommerziell verfügbar. Eine Aufzählung aller möglichen Verfahren würde den Rahmen dieser Anmeldeschrift sprengen.
Monomere für den Einbau von Struktureinheiten gemäß Formel (XIII) können beispielsweise gemäß R. H.Mitchell et al., *Org. Prep. Proced. Int.* **1997,** *29*, 715 synthetisiert werden.
Halogenierte Monomere für den Einbau von Struktureinheiten gemäß Formel (XIV) sind in großer Breite in der Literatur bekannt und teilweise sogar kommerziell verfügbar. Eine Aufzählung aller möglichen Verfahren würde den Rahmen dieser Anmeldeschrift sprengen.
Monomere für den Einbau von Struktureinheiten gemäß Formel (XV) können beispielsweise gemäß H. M. Gilow et al., *J. Org. Chem.* **1981,** *46*, 2221 und G. A. Cordell, *J. Org. Chem.* **1975,** *40*, 3161 synthetisiert werden.
Monomere für den Einbau von Struktureinheiten gemäß Formel (XVI) können beispielsweise gemäß M. A. Keegstra et al., *Synth. Commun.* **1990,** *20*, 3371 und R. Sornay et al., *Bull.* Soc. *Chim. Fr.* **1971,** *3*, 990 synthetisiert werden und sind teilweise auch kommerziell verfügbar.

Monomere für den Einbau von Struktureinheiten gemäß Formel (XVII) sind teilweise kommerziell verfügbar.
Monomere für den Einbau von Struktureinheiten gemäß Formel (XVIII) können beispielsweise gemäß JP 63-250385 synthetisiert werden.
Monomere für den Einbau von Struktureinheiten gemäß Formel (XIX) können beispielsweise gemäß M. El Borai et al., *Pol. J. Chem.* **1981**, *55*, 1659 synthetisiert werden und sind teilweise auch kommerziell verfügbar.
In den hier aufgeführten Literaturstellen zur Synthese von Monomeren, die im Polymer Strukturen gemäß den Formeln (III) bis (XIX) geben, wird hauptsächlich die Synthese von Halogenderivaten, bevorzugt von Brom-derivaten beschrieben. Davon ausgehend, ist es für den Fachmann leicht z.B. Boronsäurederivate bzw. Stannate herzustellen. Dies kann beispielsweise durch Metallierung (z. B. mit Mg (Grignard-Reaktion) oder Li (z. B. durch Bu-Li)) und anschließender Umsetzung mit entsprechenden Bor- oder Zinnderivaten, wie z. B. Borsäuretrialkylestern oder Trialkylzinnhalogeniden geschehen. Es ist aber natürlich auch möglich, Boronsäurederivate aus den entsprechenden Bromiden unter Übergangsmetall-Katalyse und Einsatz von Diboranen zu erzeugen. Weitere literaturbekannte Verfahren sind sehr vielfältig und können vom Fachmann natürlich ebenso verwendet werden.

Strukturelemente, die Etektronentransporteigenschaften aufweisen, sind beispielsweise Pyridinderivate, Pyrimidinderivate, Pyridazinderivate, Pyrazinderivate, Oxadiazolderivate, Chinolinderivate, Chinoxalinderivate, Phenazinderivate und weitere O, S oder N-haltige Heterocyclen, mit niedrigliegendem LUMO (LUMO = niedrigstes unbesetztes Molekülorbital); bevorzugt haben diese Heterocyclen ein LUMO von mehr als 2.7 eV (gegen Vakuumlevel) , besonders bevorzugt von mehr als 3.0 eV.

Bevorzugt sind dabei erfindungsgemäße Polymere, die noch mindestens eine Struktrureinheit gemäß den Formeln (XX) bis (XXX) enthalten. Der Anteil dieser Strukturelemente ist dabei mindestens 1%, bevorzugt mindestens 5%. Der maximale Anteil ist dabei höchstens 70%, bevorzugt höchstens 50%. Auch diese Struktureinheiten können im Polymer statistisch, alternierend oder blockartig eingebaut sein. 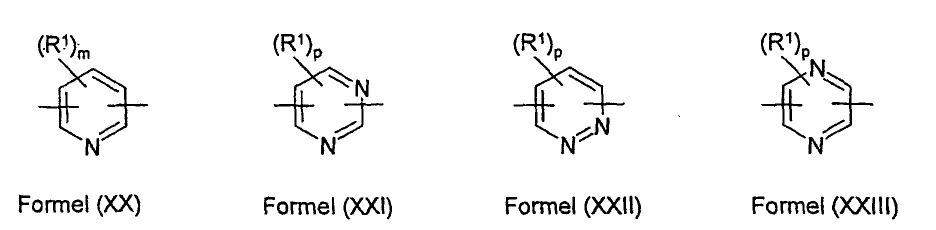 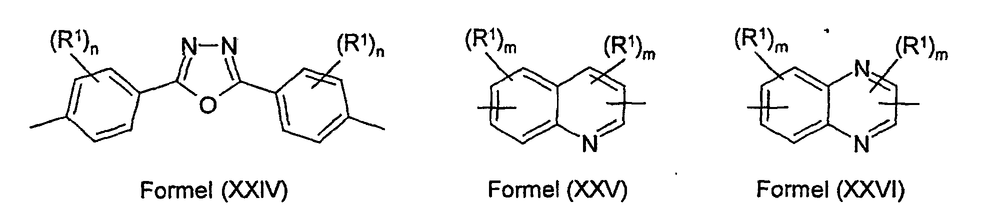 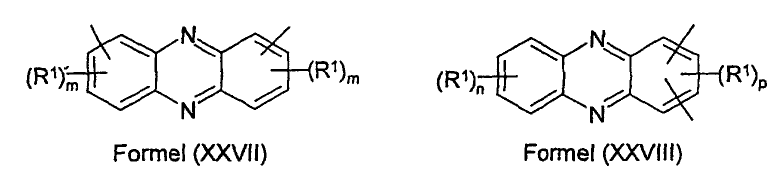 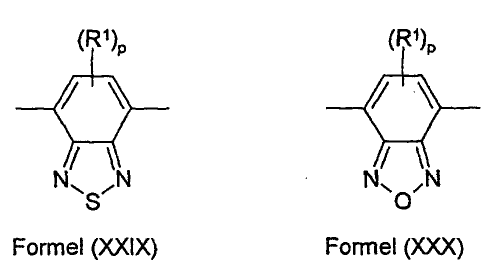 wobei das Symbol R¹ und die Indizes n und m die unter Formel (I) und Formel (II) genannten Bedeutungen haben, und der Index
- p: 0, 1 oder 2, bevorzugt 0 oder 1 bedeutet.

Die Art des Einbaus dieser Strukturen ist bei etlichen schon direkt vorgegeben (siehe z. B. Formeln (XXIV), (XXIX) und (XXX)). Bei anderen Strukturen sind jeweils mehrere Möglichkeiten erfindungsgemäß. Allerdings gibt es bei diesen auch bevorzugte Einbauweisen:
So ist bei Pyridinderivaten die Verknüpfung via 2,5- bzw. 2,6-Position, bei Pyrazinund Pyrimidinderivaten diejenige via 2,5-Position und bei Pyridazinderivaten diejenige via 3,6-Position bevorzugt.
Bei den bicyclischen Heterocyclen sind in der Regel mehrere Verknüpfungen möglich und auch bevorzugt. Bei Chinoxalin ist jedoch diejenige via 5,8-Position eindeutig bevorzugt.
Bei Phenazin kann es nun - wie angedeutet - sowohl bevorzugt sein, daß die Verknüpfung via die beiden äußeren Ringe erfolgt, oder daß nur an einem Ring eingebaut wird. Bevorzugte Positionen sind dadurch der Einbau am 1,4- bzw. 2,3-bzw. 2,7- bzw. 3,7-Kohlenstoffatom.

Die Chemie von Pyridinderivaten (XX) ist sehr ausführlich untersucht. So ist die Darstellung von 2,5- und 2,6-Dihalogenpyridinen ebenfalls bekannt. Es sei hier auf die zahlreichen Standardwerke der Heterocyclen Chemie verwiesen. Darüber hinaus sind etliche der Verbindungen auch kommerziell verfügbar.
Monomere für den Einbau von Struktureinheiten gemäß Formel (XXI) können beispielsweise gemäß Arantz et al., *J. Chem. Soc. C* **1971**, *1889* synthetisiert werden.
Monomere für den Einbau von Struktureinheiten gemäß Formel (XXII) können beispielsweise gemäß Pedrali et al., *J. Org. Synth.* **1958**, *23*, 778 synthetisiert werden.
Monomere für den Einbau von Struktureinheiten gemäß Formel (XXIII) können beispielsweise gemäß Ellingson et al., *J. Am. Chem. Soc.* **1949**, *71*, 2798 synthetisiert werden.
Monomere für den Einbau von Struktureinheiten gemäß Formel (XXIV) können beispielsweise gemäß Stolle ef al., *J. Prakt. Chem.* **1904**, *69*, 480 synthetisiert werden.
Monomere für den Einbau von Struktureinheiten gemäß Formel (XXV) können beispielsweise gemäß Metzger, *Chem. Ber.* **1884,** *17*,187 und A. I. Tochilkin et al., *Chem. Heferocycl. Compd. (Engl. Transl)* **1988,** 892 synthetisiert werden.
Monomere für den Einbau von Struktureinheiten gemäß Formel (XXVI) können beispielsweise gemäß Calhane et al., *J. Am. Chem. Soc.* **1899,** *22*, 457 und T. Yamamoto et al., *J. Am. Chem. Soc.* **1996**, *118*, 3930 synthetisiert werden.
Monomere für den Einbau von Struktureinheiten gemäß Formel (XXVII) und (XXVIII) können beispielsweise gemäß L. Horner et al., *J. Liebigs Ann. Chem.,* **1955,** *597*, 1 und P. R. Buckland et al., *J. Chem. Res. Miniprint* **1981,** *12*, 4201 synthetisiert werden. Monomere für den Einbau von Struktureinheiten gemäß Formel (XXIX) können beispielsweise gemäß K. Pilgram et al., *J. Heterocycl. Chem.* **1970,** *7*, 629 und WO 00/55927 synthetisiert werden.
Monomere für den Einbau von Struktureinheiten gemäß Formel (XXX) können beispielsweise gemäß Hammick et al., *J. Chem. Soc.* **1931,** 3308 und K. Pilgram et al., *J. Heterocycl. Chem.* **1974,** *11*, 813 synthetisiert werden.
Auch in den hier aufgeführten Literaturstellen zur Synthese von Monomeren, die im Polymer Strukturen gemäß den Formeln (XX) bis (XXX) geben, wird hauptsächlich die Synthese von Halogenderivaten, bevorzugt von Brom-derivaten beschrieben. Davon ausgehend, kann der Fachmann, wie auch oben bei den die Lochmobilität erhöhenden Einheiten beschrieben, weitere Umwandlungen, z. B. zu Boronsäurederivaten oder Stannaten, vornehmen.

Bevorzugt sind weiterhin auch erfindungsgemäße Polymere, bei denen sowohl Einheiten, welche die Lochbeweglichkeit, als auch solche, welche die Elektronenbeweglichkeit erhöhen, enthalten sind.
Besonders bevorzugt sind demgemäß erfindungsgemäße Polymere, die sowohl ein oder mehrere Strukturen gemäß den Formeln (III) bis (XIX), als auch ein oder mehrere Strukturen gemäß den Formeln (XX).bis (XXX) enthalten.
Dabei sollen weiterhin die o. g. Grenzen für den jeweiligen Anteil gelten.
Es kann dabei ganz besonders bevorzugt sein, daß man in den erfindungsgemäßen Polymeren Einheiten hat, in denen Lochbeweglichkeit- und Elektronenbeweglichkeiterhöhende Strukturen direkt aufeinanderfolgen bzw. sich abwechseln, wie dies beispielsweise für die Formeln (XXXI) bis (XXXXV) der Fall ist:

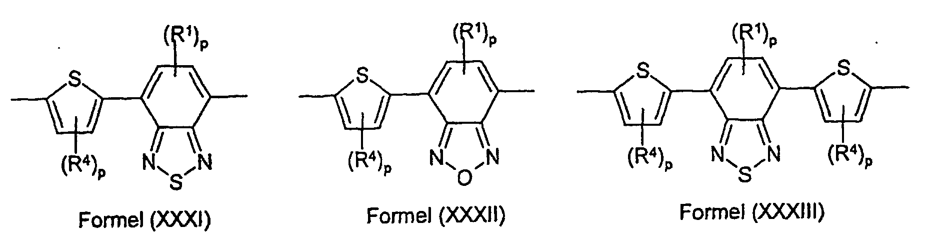

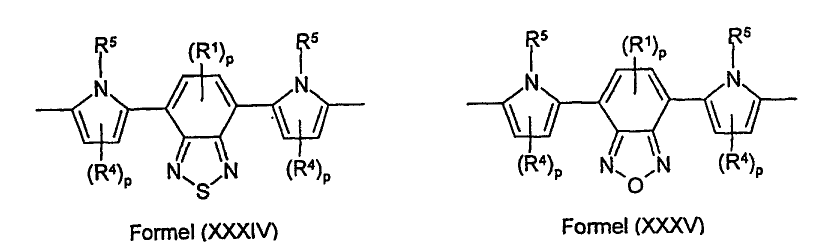

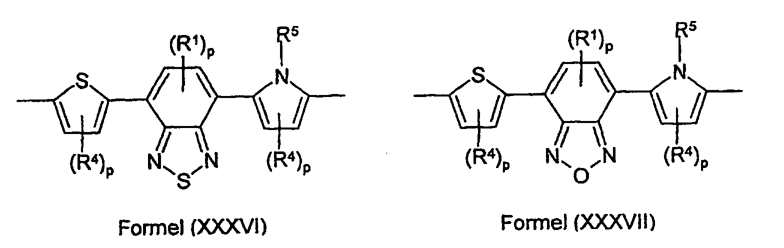

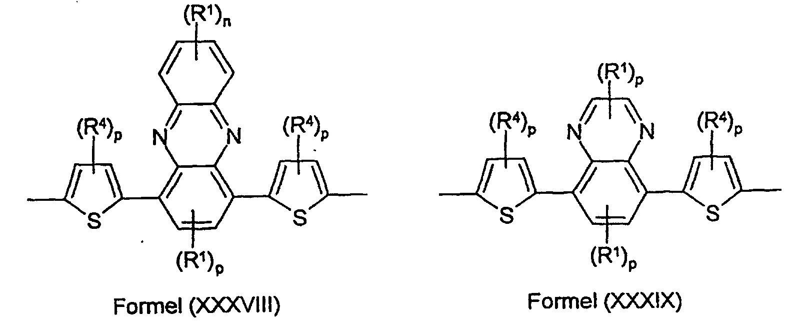

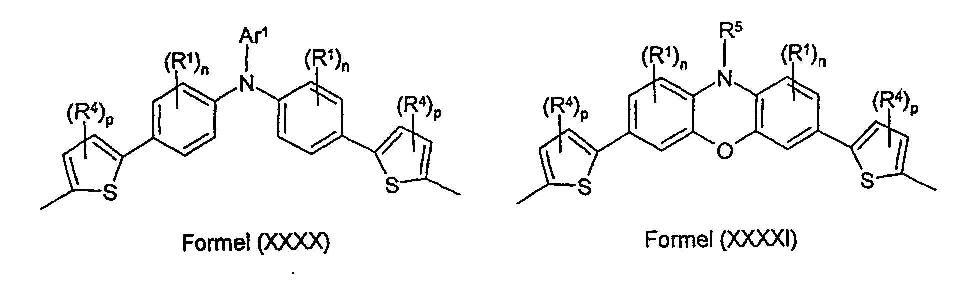

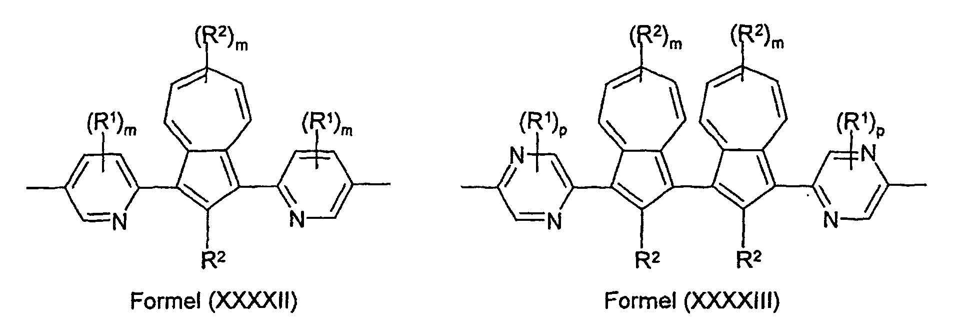

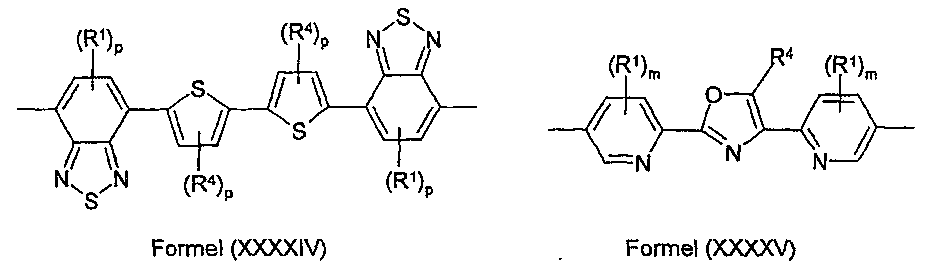

Monomere gemäß den Formeln (XXXl) bis (XXXXV) lassen sich gemäß den für die Formeln (III) bis (XXX) gemachten Angaben durch entsprechende Kombination der entsprechenden Vorstufen synthetisieren. Es sei auch darauf verwiesen, daß zumindest einige Synthesebeispiele in den oben bereits genannten Anmeldungen WO 00/46321 und WO 00/55927 aufgeführt sind. Weiterhin wird auch beispielsweise in H. A. M. Mullekom et al., *Chem. Eur. J.*, **1998**, *4*, 1235 von derartigen Strukturen berichtet. Es sei darauf verwiesen, daß die Strukturen gemäß den Formeln (XXXI) bis (XXXXV) keineswegs die Erfindung darauf begrenzen soll, sondern daß es natürlich für den Fachmann ein leichtes ist, aus den o. g. Strukturen (III) bis (XIX) bzw. (XX) bis (XXX) geeignete Kombinationen zu synthetisieren und diese in die erfindungsgemäßen Polymere zu inkorporieren.

Bevorzugte Copolymere, die noch weitere Strukturelemente neben denen gemäß Formel (I) und Formel (II) enthalten, sind des weiteren solche, die mindestens noch eine weitere aromatische oder andere eine konjugierte Struktur aufweisen, welche nicht unter die o. g. Gruppen fällt; d. h. die die Ladungsträgermobilitäten nicht oder nur wenig beeinflußt. Derartige Strukturelemente können die Morphologie, aber auch in besonderem Maße die Emissionsfarbe der resultierende Polymere beeinflußen.

Bevorzugt sind dabei aromatische Strukturen, die 6 bis 40 C-Atome aufweisen oder auch Stilben- oder Bisstyrylarylenderivate, die jeweils mit einem oder mehreren nicht aromatischen Resten R¹ substituiert sein können.
Besonders bevorzugt ist dabei der Einbau von 1,4-Phenylen-, 1,4-Naphthylen-, 1,4-oder 9,10-Anthracenylen-, 1,6- oder 2,7- oder 4,9-Pyren-, 3,9- oder 3,10- Perylen-, 2,7- oder 3,6-Phenanthren-, 4,4'-Biphenylen-, 4,4"-Terphenylen-, 4,4'-Bi-1,1'naphthylen-, 4,4'-Stilben- oder 4,4"-Bisstyrylarylenderivate.
Derartige Strukturen sind vielfach in der Literatur bekannt und zum großen Teil auch kommerziell verfügbar. Eine Aufführung aller möglichen Synthesevarianten würde den Rahmen dieser Anmeldeschrift deutlich sprengen.

Bevorzugte Copolymere, die noch weitere Strukturelemente neben denen gemäß Formel (I) und Formel (II) enthalten, sind des weiteren auch noch solche, die metallorganische Komplexe in die Hauptkette inkorporiert haben. Besonders bevorzugt sind dabei Komplexe der d-Übergangsmetalle, ganz besonders solche der höheren Metalle der Eisen-, Cobalt- und Nickeltriade, d. h. Komplexe von Ruthenium, Osmium, Rhodium, Iridium, Palladium und Platin. Derartige Komplexe sind häufig in der Lage, aus angeregten Triplett-Zuständen Licht zu emittieren, was häufig eine Steigerung der Energieeffizienz bewirkt. Die Verwendung derartiger Komplexe in niedermolekularen OLEDs ist beispielsweise in M. A. Baldo, S. Lamansky, P. E. Burrows, M. E. Thompson, S. R. Forrest, Applied Physics Letters, **1999,** *75*, 4-6 beschrieben. Über den Einbau dieser Verbindungen in Polymere wurde bislang noch nichts berichtet. Entsprechende Monomere sind in der noch nicht offengelegten Anmeldeschrift DE 10109027.7 beschrieben. Derartige Strukturelemente können die Morphologie aber auch in besonderem Maße die Emissionsfarbe und die Energieeffizienz der resultierende Polymere beeinflussen.

Als Beispiele für besonders bevorzugte Komplexe, die in erfindungsgemäße Polymere eingebaut werden können, seien hier die Verbindungen gemäß den Formeln (XXXXVl) bis (XXXXlX) genannt. 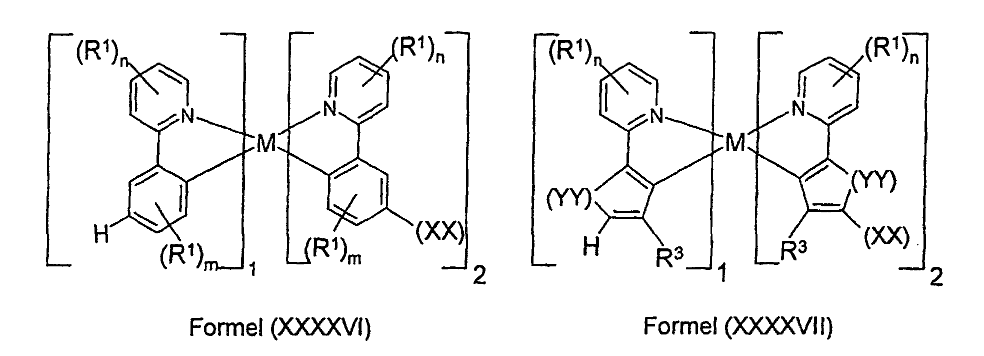 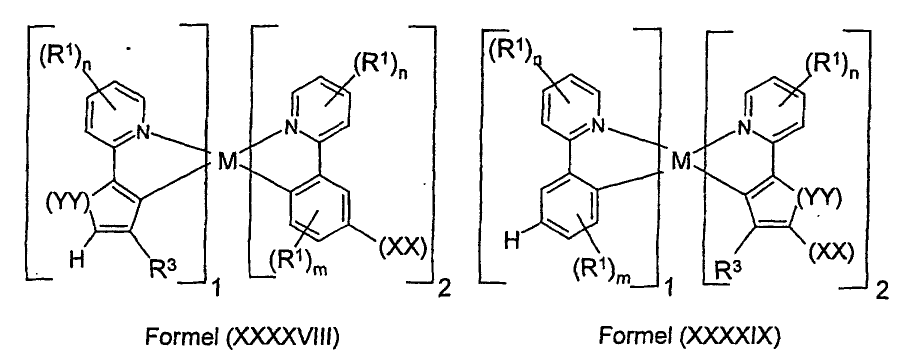 In diesen Formeln haben die Symbol R¹ und R³ und die Indices n und m die unter den Formeln (I) und (II) genannten Bedeutungen.
- M: entspricht Rh oder lr
- XX: entspricht der Verknüpfungsstelle im Polymer
- YY: ist bei jedem Auftreten gleich oder verschieden O, S oder Se.

Die Herstellung entsprechender Monomere ist in der o. g. nicht offengelegten Anmeldeschrift DE 10109027.7 ausgeführt.

Die erfindungsgemäßen Polymere werden nun in der Regel durch Polymerisation von zwei oder mehreren Monomeren, von den mindestens eines anschließend Strukturen der Formel (I) und mindestens ein weiteres solche der Formel (II) ergibt, hergestellt.
Entsprechende Polymerisationsreaktionen gibt es prinzipiell relativ viele Verschiedene, es haben sich jedoch die im folgenden aufgeführten Typen besonders bewährt. Grundsätzlich ergeben all diese Reaktionstypen C-C-Verknüpfungen:
(A) Polymerisation gemäß SUZUKI: Hierbei werden als Monomere zum einen Bishalogenide, zum anderen Bisboronsäuren und entsprechende -derivate, oder entsprechende Monohalogenid-monoboronsäurederivate, eingesetzt und unter Palladiumkatalyse, in Anwesenheit von Lösemitteln und basischen Bedingungen gekuppelt. Derartige Reaktionen, welche zu konjugierten Polymeren führen, sind bereits vielfach beschrieben. Es gibt eine ganze Reihe von Vorschlägen, wie derartige Reaktionen effizient ablaufen und zu hochmolekularen Polymeren führen; diese sind u. a. in den folgenden Stellen aufgeführt: (i) EP 707.020, (ii) EP 842.208, (iii) EP 1.025.142, (iv) WO 00/53656, und (v) in den darin zitierten Literaturstellen. Die entsprechenden Beschreibungen werden hiermit via Zitat als Bestandteil der Anmeldung erachtet.
(B) Polymerisationen gemäß YAMAMOTO: Hierbei werden als Monomere ausschließlich Bishalogenide verwendet. Diese werden in Anwesenheit von Lösemitteln, einer Nickel-verbindung, einer Base und gegebenenfalls eines Reduktionsmittels sowie weiterer Liganden durchgeführt. Derartige Reaktionen, welche zu konjugierten Polymeren führen, sind bereits öfters beschrieben. Es gibt einige Vorschläge, wie derartige Reaktionen effizient ablaufen und zu hochmolekularen Polymeren führen; diese sind u. a. in den folgenden Stellen aufgeführt: (i) M. Ueda et al., *Macromolecules,* **1991,** *24*, 2694, (ii) T. Yamamoto et al., *Macromolecules* **1992**, *25*, 1214, (iii) T. Yamamoto et al., *Synth. Met.* **1995,** *69*, 529-31, (iv) T. Yamamoto et al., *J. Organometallic Chem.* **1992,** *428*, 223, (v) l. Colon et al., *J. Poly. Sci.: Part A: Poly. Chem.* **1990,** *28*, 367, (vi) T. Yamamoto et al., *Macromol. Chem. Phys.* **1997,** *198*, 341. Die entsprechenden Beschreibungen werden hiermit via Zitat als Bestandteil der Anmeldung erachtet.
(C) Polymerisationen gemäß STILLE: Hierbei werden als Monomere zum einen Bishalogenide, zum anderen Bisstannate, oder entsprechende Monohalogenidmonostannate, eingesetzt und unter Palladiumkatalyse, in Anwesenheit von Lösemitteln und basischen Bedingungen gekuppelt. Derartige Reaktionen, welche zu konjugierten Polymeren führen, sind bereits beschrieben. Es gibt hier allerdings noch nicht so weite Ausarbeitungen, wie dies im Falle der SUZUKIoder YAMAMOTO-Kupplung der Fall ist. Ein konjugiertes Polymer, welches. durch STILLE-Kupplung erhalten wurde, wird z. B. in W. Schorf et al., *J. Opt. Soc. Am. B* **1998,** *15*, 889 beschrieben. Eine Übersicht über die Möglichkeiten und die Schwierigkeiten der STILLE-Reaktion gibt u. a. V. Farina, V. Krishnamurthy, W. J. Scott (Hers.) **"The Stille Reaction" 1998,** Verlag: Wiley, New York, N. Y. Die entsprechenden Beschreibungen werden hiermit via Zitat als Bestandteil der Anmeldung erachtet.

Nach der durchgeführten Polymerisation (Polykondensation) müssen die synthetisierten Polymere zunächst vom Reaktionsmedium abgetrennt werden. Dies geschieht in der Regel durch Ausfällen in einem Nicht-Lösemittel. Anschließend müssen die erhaltenen Polymere aufgereinigt werden, da gerade der Gehalt an organischen niedermolekularen Verunreinigungen und auch der lonengehalt bzw. Gehalt an sonstigen anorganischen Verunreinigungen teilweise sehr starke Auswirkungen auf die Anwendungseigenschaften der Polymere in PLEDs haben. So können niedermolekulare Bestandteile zum einen die Effizienz erheblich absenken, aber auch die operative Lebensdauer dramatisch verschlechtern. Analoges gilt für die Anwesenheit von anorganischen Verunreinigungen.
Geeignete Reinigungsverfahren sind zum einen Umfällvorgänge, bei denen das Polymer mehrfach gelöst und in einem Nicht-Lösemittel gefällt wird. Es ist dabei sinnvoll, die Polymerlösung über einen Filter zu geben, um von ungelösten Bestandteilen (vemetzten Gelpartikel) und auch Staubpartikeln abzutrennen. Eine weitere Möglichkeit ist das Verwenden von Ionenaustauschern, um den Gehalt an lonen zu erniedrigen. Hierbei kann auch das Ausrühren einer Polymerlösung mit einer wässrigen Lösung, welche z. B. chelatisierende Liganden enthält, helfen. Auch weitere organische oder anorganische Extraktionsverfahren, z. B. mit Lösemittel / Nicht-Lösemittelgemischen, oder mit überkritischem CO₂ können hier deutliche Verbesserungen bringen.

Die so erhaltenen erfindungsgemäßen Polymere können nun in PLEDs verwendet werden. Dazu wird in der Regel folgendes allgemeine Verfahren verwendet, daß natürlich für den Einzelfall dann entsprechend anzupassen ist:
- Ein Substrat (z. B. Glas oder auch ein Kunststoff, wie speziell behandeltes PET) wird mit einem transparenten Anodenmaterial beschichtet (beispielsweise Indium-Zinn-Oxid, ITO); anschließend wird die Anode (z. B. photolithografisch) der gewünschten Anwendung gemäß strukturiert und verschaltet. Es kann hier auch sein, daß das ganze Substrat und die entsprechende Verschalung zunächst über einen recht aufwendigen Prozeß erzeugt wird, um dadurch eine sogenannte Aktiv-Matrix-Steuerung zu ermöglichen.
- Anschließend wird entweder vollflächig, oder nur auf die aktiven (= anodischen) Stellen i. d. R. zunächst ein leitfähiges Polymer, z. B. ein dotiertes Polythiophenoder Polyanilinderivat, aufgebracht. Dies erfolgt in aller Regel durch Beschichtungsverfahren, welche eine Dispersion des entsprechenden Polymers aufbringen. Hierzu eignen sich prinzipiell die weiter unten für das lichtemittierende Polymer beschriebenen Verfahren. Die Schichtdicke dieser Polymerlage kann in weiten Bereichen variieren, wird aber für die praktische Anwendung im Bereich zwischen 10 und 1000 nm, bevorzugt zwischen 20 und 500 nm liegen.
- Darauf bringt man dann, je nach Anwendung, eine Lösung eines erfindungsgemäßen Polymers auf. Für mehr- oder vollfarbige Anzeigeelemente (Displays) werden dann mehrere verschiedene Lösungen in verschiedenen Regionen aufgebracht, um entsprechende Farben zu erzeugen. Die erfindungsgemäßen Polymeren werden dazu zunächst einzeln (es kann auch empfehlenswert sein, Blends von zwei oder mehr Polymeren zu verwenden) in einem Lösemittel oder Lösemittelgemisch gelöst und schließlich filtriert. Da die organischen Polymere und v. a. die Zwischenschichten (Interface) in der PLED teilweise extrem durch Sauerstoff oder andere Luftbestandteile beeinflußt werden, empfiehlt es sich, diese Operation unter Schutzgas durchzuführen. Als Lösemittel eignen sich aromatische Flüssigkeiten wie beispielsweise Toluol, Xylole, Anisol, Chlorbenzol, aber auch andere, wie beispielsweise cyclische Ether (z. B. Dioxan, Methyldioxan) oder auch Amide, wie beispielsweise NMP oder DMF, aber auch Lösemittelgemische, wie diese in der nicht offengelegten Anmeldeschrift DE 10111633.0 beschrieben werden. Mit diesen Lösungen können nun die vorher beschriebenen Träger beschichtet werden, und zwar entweder ganzflächig z. B. durch Spin-Coat-Verfahren oder Rackel-Techniken, oder aber auch aufgelöst durch Druckverfahren, wie Tintenstrahldrucken, Off-Set-Drucken, Screen-Printing-Verfahren, Tiefdruckverfahren, und ähnlichen.
- Auf diese Polymerschichten können nun wahlweise noch Elektroneninjektionsmaterialien aufgebracht werden, z. B. durch Aufdampfen, oder auch aus Lösung, durch Methoden, wie diese für die emittierenden Polymere beschrieben wurden. Als Elektroneninjektionsmaterialien können beispielsweise niedermolekulare Verbindungen, wie Triarylboranverbindungen oder auch Aluminium-trishydroxychinolinat (Alq₃), aber auch entsprechende Polymere, wie beispielsweise Poly-pyridinderivate und ähnliche, verwendet werden. Es ist auch möglich, dünne Schichten des emittierenden Polymers durch entsprechendes Dotieren, zu Elektroneninjektionsschichten umzuwandeln.
- Daran anschließend wird eine Kathode aufgedampft. Dies erfolgt i. d. R. durch einen Vakuumprozeß und kann beispielsweise sowohl durch thermisches Bedampfen als auch durch Plasmaspritzen (Sputtern) geschehen. Die Kathode kann dabei vollflächig oder auch durch eine Maske strukturiert aufgebracht werden. Als Kathode werden i. d. R. Metalle mit geringer Austrittsarbeit, z. B. Alkali-, Erdalkali- und f-Übergangsmetalle, wie z. B. Ca, Mg, Sr, Ba, Yb, Sm, oder auch Aluminium, oder auch Legierungen von Metallen, oder auch mehrlagige Strukturen mit verschiedenen Metallen verwendet. Bei letzterem können auch Metalle mitverwendet werden, die eine relativ hohe Austrittsarbeit aufweisen, wie z. B. Ag. Es kann auch bevorzugt sein, zwischen das Metall und das emittierende Polymer bzw. die Elektroneninjektionsschicht, eine sehr dünne dielektrische Schicht (z. B. LiF oder ähnliches) einzubringen. Die Kathoden sind i. d. R. zwischen 10 und 10000 nm, bevorzugt zwischen 20 und 1000 nm, dick.
- Anschließend werden die so erzeugten PLEDs bzw. Displays entsprechend angeschlossen und verkapselt um dann getestet oder verwendet zu werden.

Wie oben beschrieben, eignen sich die erfindungsgemäßen Polymere ganz besonders als Elektroluminszenzmaterialien in den derart hergestellten PLEDs oder Displays.

Als Elektrolumineszenzmaterialien im Sinne der Erfindung gelten Materialien, die als aktive Schicht in einer PLED Verwendung finden können. Aktive Schicht bedeutet, daß die Schicht befähigt ist, bei Anlegen eines elektrischen Feldes Licht abzustrahlen (lichtemittierende Schicht) und/oder daß sie die Injektion und/oder den Transport der positiven und/oder negativen Ladungen verbessert (Ladungsinjektions- oder Ladungstransportschicht).

Gegenstand der Erfindung ist daher auch die Verwendung eines erfindungsgemäßen Polymers in einer PLED, insbesondere als Elektrolumineszenzmaterial.

Gegenstand der Erfindung ist somit ebenfalls eine PLED mit einer oder mehreren aktiven Schichten, wobei mindestens eine dieser aktiven Schichten ein oder mehrere erfindungsgemäße Polymere enthält. Die aktive Schicht kann beispielsweise eine lichtemittierende Schicht und/oder eine Transportschicht und/oder eine Ladungsinjektionsschicht sein.

PLEDs finden z.B. Anwendung als selbstleuchtende Anzeigeelemente, wie Kontrollampen, alphanumerische Displays, mehr- oder vollfarbigen Displays, Hinweisschilder, und in optoelektronischen Kopplern.

Im vorliegenden Anmeldetext und auch in den im weiteren folgenden Beispielen wird auf die Verwendung erfindungsgemäßer Polymere oder Blends aus erfindungsgemäßen Polymeren in Bezug auf PLEDs und den entsprechenden Displays abgezielt. Trotz dieser Beschränkung der Beschreibung ist es für den Fachmann ohne weiteres erfinderisches Zutun möglich, die erfindungsgemäßen Polymere auch für weitere Verwendungen in anderen elektronischen Devices (Vorrichtungen) zu benutzen, z. B. für Organische Integrierte Schaltungen (O-lCs), in Organischen Feld-Effekt-Transistoren (OFETs), in Organischen Dünnfilmtransistoren (OTFTs), für Organische Solarzellen (O-SCs) oder auch Organische Laserdioden (O-Laser), um nur einige Anwendungen zu nennen.

Die Erfindung wird durch die nachfolgenden Beispiele näher erläutert.

### Teil A: Synthese der Monomere:

### A1: Monomere für Einheiten gemäß Formel (I) (Spiro-Verbindungen)

Beispiel M1/M2: Herstellung von 2,7-Dibromo-2',3',6',7'-tetra(2-methylbutyloxy)spirobifluoren und 2',3',6',7'-Tetra(2-methylbutyloxy)spirobifluoren-2,7-bisboronsäureethyfenglycolester

### Darstellung von 3,3',4,4'-Tetra(2-methylbutyloxy)biphenyl

229.405 g (696.6 mmol) 3,4-Bis(2-methylbutyloxy)-1-brombenzol, 215 g (731 mmol) 3,4-Bis(2-methyl-butyloxy)benzolboronsäure und 202.1g (1.462mol) K₂CO₃ wurden in 800 ml Toluol und 800 ml Wasser suspendiert und es wurde 1 h mit N₂ gesättigt. Anschließend wurde unter Schutzgas 1.74 g (1.505 mmol) Pd(PPh₃)₄ zugegeben. Das trübe, leicht gelbliche Gemisch wurde unter Stickstoff bei Rückfluß für ca. 7h kräftig gerührt. Nach Abkühlen wurde die organische Phase mit 500 ml 1%-iger NaCN Lsg. ausgerührt. Es wurde mit Wasser nachgewaschen, mit Na₂SO₄ getrocknet und einrotiert. Man erhielt 339 g (679.7 mmol, 98%) eines leicht braunen Öls, welches It. ¹H NMR 97%ig war und direkt in die Folgereaktion eingesetzt wurde. ¹H NMR (CDCl₃, 500 MHz): 7.05 (s, 2 H, H2/H2'); 7.04 (d, 2 H, H6/H6', J = 8.5); 6.91 (d, 2H, H5/H5', J = 8.6); 3.94-3.77 (m, 8H, OCH₂); 1.98-1.82 (m, 4H, H-C); 1.68-1.58 (m, 4H, CH₂); 1.39-1.25 (m, 4H, CH₂); 1.07-0.93 (m, 24 H, 8 x CH₃).

### Herstellung von 2-Bromo-4,5,3',4'-tetra(2-methylbutyloxy)biphenyl

339 g (679.7) mmol 3,3',4,4'-Tetra(2-methylbutyloxy)biphenyl wurden in 800ml Essigester gelöst. Dann wurde unter Schutzgas, Lichtausschluß und Kühlung auf 0-5°C 120.98 g (679.7 mmol) N-Bromsuccinimid innerhalb von 15 Minuten als Feststoff zugegeben. Die Suspension wurde unter Schutzgasüberlagerung langsam auf Raumtemperatur erwärmt und dann bei Raumtemperatur für 4 Stunden kräftig gerührt. Es wurden 500 ml Essigester und 300 ml Wasser zugefügt, die Phasen getrennt und die wäßrige Phase noch zweimal mit je 100 ml Essigester extrahiert.

Die vereinigten organischen Phasen wurden zweimal mit je 50 ml Wasser gewaschen und mit MgSO₄ getrocknet. Das erhaltene Öl wurde mit Hexan durch Kieselgel filtriert. Man erhielt nach Abziehen des Lösungsmittels 361.2 g (625.3 mmol, 92%) 2-Bromo-4,5,3',4'-tetra(2-methylbutyloxy)biphenyl als farbloses Öl. ¹H NMR (CDCl₃, 500 MHz): 7.10 (s, 1 H, H6); 6.93 (d, 1 H, H6', J = 1.3 Hz); 6.88 (s, 1H, H3); 6.87 (d, 1H, H5', J = 1.4 Hz); 6.38 (s, 1 H, H2'); 3.92-3.73 (m, 8H, OCH₂); 1.98-1.84 (m, 4H, H-C); 1.68-1.54 (m, 4H, CH₂); 1.37-1.25 (m, 4H, CH₂); 1.08-0.91 (m, 24 H, 8 x CH₃).

### Herstellung von 2,7-Dibromo-2',3',6',7'-tetra(2-methylbutyloxy)spirobifluoren (M1)

360 g (623.2 mmol) 2-Bromo-4,5,3',4'-tetra(2-methylbutyloxy)biphenyl wurden in 400 ml destilliertem THF gelöst. 15.59 g (641.89 mmol, 1.03eq) Magnesiumspäne sowie einige Kristalle lod wurden unter N₂ vorgelegt. Es wurde kurz erhitzt und 10% der Eduktmenge in THF wurde zugegeben. Nach dem Anspringen der Reaktion gab man den Rest so zu, daß das Reaktionsgemisch ohne weitere Wärmezuführung von selbst am Rückfluß siedete (eine Stunde). Man ließ weitere 2 Stunden refluxieren und gab danach noch weitere 100 ml destilliertes THF zu.
Eine Suspension von 210.64 g (623.2 mmol) 2,7-Dibromfluoren-9-on in 800 ml destilliertem THF wurde auf 0°C abgekühlt. Die Grignardlösung wurde nun bei einer Temperatur von 0-5°C zu der Suspension getropft. Anschließend wurde für 90 Minuten unter Rückfluß erhitzt.
Nach.Abkühlen auf Raumtemperatur wurde zu dem Reaktionsgemisch eine Mischung aus 755 ml Eiswasser und 41.7ml HCl (37%ig) gegeben und 30 Minuten gerührt. Die organische Phase wurde zuerst mit NaHCO₃ Lösung (2 x 30 ml), danach mit Wasser (2 x 100 ml) gewaschen, über Natriumsulfat getrocknet und einrotiert. Man erhielt 530 g eines hellbraunes Öls, weiches sofort weiter umgesetzt wurde.
Es wurde mit 1250 ml Essigsäure und 18.5 ml konzentrierter Salzsäure versetzt und unter Stickstoff zum Sieden erhitzt. Nach 10 Minuten wurde noch 200 ml Essigsäure zugegeben. Nach 2 Stunden wurde auf Raumtemperatur abgekühlt, der ausgefallene Feststoff wurde abfiltriert und erst mit 200 ml Essigsäure, dann mit 200 ml Wasser gewaschen und im Vakuum getrocknet. Der Feststoff wurde mit Methanol ausgerührt und über Nacht bei 40°C im Trockenschrank getrocknet.

Der Feststoff wurde 2 x aus 2-Butanon umkristallisiert. Man erhielt 329.7 g (402.7 mmol, 64%) 2,7-Dibromo-2',3',6',7'-tetra(2-methylbutyloxy)spirobifluoren als farblosen Feststoff, welcher It. HPLC (Zorbax SB-C18 3.5µm, 4.6 x 75mm, 90% Methanol/10% THF, 1ml/min, UV Detektion 230-330 nm) eine Reinheit von >99.8% aufwies.
¹H NMR (CDCl₃, 400 MHz): 7.64 (d, 2 H, H4/5, J = 8.0 Hz); 7.47 (dd, 2 H, H3/6, J = 8.0, 1.9 Hz); 7.19 (d, 2H, H1/8, J ~ 1.5 Hz); 6.85 (s, 2H, H4'/5'); 6.12 (s, 2H, H1'/8'); 3.97-3.83 (m, 4H, 2 x OCH₂); 3.58-3.45 (m, 4H, 2 x OCH₂); 2.02-1.89 (m, 2H, H-C); 1.80-1.70 (m, 2H, H-C); 1.68-1.59 (m, 2H, CH₂); 1.53-1.42 (m, 2H, CH₂); 1.39-1.26 (m, 2H, CH₂); 1.22-1.10 (m, 2H, CH₂); 1.08 (d, 6H, 2 x CH₃, J = 6.7 Hz); 0.95 (ϕt, 6H, 2 x CH₃, J = 7.4 Hz); 0.93 (d, 6H, 2 x CH₃, J = 6.9 Hz); 0.86 (ϕt, 6H, 2 x CH₃, J = 7.4 Hz).

### Herstellung von 2',3',6',7'-Tetra(2-methylbutyloxy)spirobifluoren-2,7-bisboronsäureethylenglycolester (M2)

150 g (183 mmol) 2,7-Dibromo-2',3',6',7'-tetra(2-methylbutyloxy)spirobifluoren wurden in 500 ml destilliertem THF gelöst. 11.2 g (458 mmol) Magnesiumspäne wurden unter Argon mit etwas lod versetzt, kurz erhitzt und mit 5% der Eduktlösung versetzt. Nachdem der Grignard angesprungen war, wurde die restliche Menge so zugetropft, daß das Lösungsmittel von selbst siedete. Nach beendeter Zugabe erhitzte man noch 3 Stunden unter Rückfluß und kühlte dann auf Raumtemperatur. Man löste 47.6 g (458 mmol, 51.2 ml) Borsäuretrimethylester in 300 ml THF und kühlte anschließend auf -78°C. Dann wurde die Grignardlösung zugetropft, wobei -60°C nicht überschritten wurde. Man ließ über Nacht auf Raumtemperatur erwärmen und versetzte die Suspension mit 250 ml Essigester. Anschließend wurden 600 g Eiswasser und 15 ml konzentrierte H₂SO₄ zugegeben und 1 Stunde gerührt. Die wäßrige Phase wurde mit 300 ml Essigester extrahiert, die vereinigten organischen Phasen wurden mit gesättigter NaCl-Lösung gewaschen, mit Na₂SO₄ getrocknet und im Vakuum eingeengt.
Der Feststoff wurde in 600 ml Chloroform gelöst, 31.7 ml (563 mmol) Ethylenglycol und 1.5 ml konzentrierte H₂SO₄ wurden zugegeben und insgesamt 10 h mit einem Wasserabscheider zum Rückfluß erhitzt. Das Lösemittel wurde im Vakuum entfernt.

Die Substanz wurde in einem Gemisch aus 60 ml Wasser und 540 ml Ethylenglycol suspendiert und eine Stunde gerührt.
Man filtrierte ab und wusch den Rückstand mit Methyl-*t*-butylether nach. Die Substanz wurde in 500 ml Methyl-*t*-butylether suspendiert und 3 Stunden zum Rückfluß erhitzt. Man ließ über Nacht stehen, filtrierte ab und wusch mit Methyl-*t*-butylether nach. Es wurde erneut in 500 ml Methyl-*t*-butylether suspendiert und 2 Stunden zum Rückfluß erhitzt. Man ließ über Nacht stehen, filtrierte ab und wusch mit Methyl-*t*-butylether nach. Man erhielt nach Trocknung im Vakuum 85.73 g (107.1 mmol, 58%) 2',3',6',7'-Tetra(2-methylbutyloxy)spirobifluoren-2,7-bisboronsäureethylen-glycolester als weißen Feststoff, welcher It. HPLC (Zorbax SB-C18 3.5µm, 4.6 x 75mm, 98% Acetonitril/2% Ethylenglycol, 1ml/min, UV Detektion 230-330 nm) eine Reinheit von > 99.8% aufwies.
¹H NMR (CDCl₃, 400 MHz): 7.56 (d, 2 H, H4/5, J = 7.4 Hz); 7.82 (d, 2 H, H3/6, J = 7.6 Hz); 7.20 (s, 2H, H1/8); 7.17 (s, 2H, H4'/5'); 6.07 (br. s, 2H, H1'/8'); 4.27 (s, 8H, Ethylenglycol); 3.97-3.85 (m, 4H, 2 x OCH₂); 3.53-3.38 (m, 4H, 2 x OCH₂); 2.00-1.89 (m, 2H, C-H); 1.92-1.60 (m, 4H, C-H, CH₂); 1.48-1.38 (m, 2H, CH₂); 1.35-1.27 (m, 2H, CH₂); 1.17-1.07 (m, 2H, CH₂); 1.07 (d, 6H, 2 x CH₃, J = 6.7 Hz); 0.97 (t, 6H, 2 x CH₃, J = 7.5 Hz); 0.93 (d, 6H, 2 x CH₃, J = 6.7 Hz); 0.82 (t, 6H, 2 x CH₃, J = 7.4 Hz).

### Herstellung von 2,7-Dibrom-2', 7'-di-tert-butyl-spirobifluoren (M3)

200 g (579.1 mmol) 2-Brom-4,4'-di-tert-butyl-biphenyl (die Herstellung dieser Verbindung ist in Org. Prep. Proced. Int. **1983**, *15*,271 sowie in J. Org. Chem. **1979**, *44*, 3037 beschrieben) wurden in 400 ml destilliertem THF gelöst. 14.55 g (598.5 mmol) Magnesiumspäne sowie einige Kristalle lod wurden vorgelegt. Es wurde kurz erhitzt und danach wurde 5% der Eduktmenge in THF zugegeben. Nach dem Anspringen der Reaktion gab man den Rest so zu, daß das Reaktionsgemisch ohne weitere Wärmezuführung von selbst am Rückfluß siedete (eine Stunde). Man ließ danach 2 Stunden refluxieren und dekantierte vom verbliebenen Magnesium ab. Man kühlte auf 0 °C ab und tropfte unter heftigem Rühren innerhalb von 40 Minuten eine Suspension von 195.75 g (579.1 mmol) 2,7-Dibromfluoren-9-on in 1 L THF zu. Das Eisbad wurde entfernt und dann wurde 90 Minuten zum Rückfluß erhitzt. Die Reaktionsmischung wurde auf 1800 g Eis mit 45 ml konz. HCl aufgegossen und gerührt bis das Eis geschmolzen war. Die organischen Phasen wurden 2 x mit je 30 ml gesättigter NaHCO₃ danach 2 x mit je 100 ml Wasser gewaschen, über Natriumsulfat getrocknet und eingeengt. Man erhielt 379 g eines hellbraunes Öls, welches direkt weiter umgesetzt wurde.
Dieses Öl wurde mit 800 ml Essigsäure und 9.0 ml konzentrierter Salzsäure unter Stickstoff zum Sieden erhitzt. Nach 2 Stunden wurde abgekühlt, der ausgefallene Feststoff wurde abfiltriert und mit Essigsäure (200 ml) und Wasser (300 ml) gewaschen. Der Feststoff wurde mit Methanol ausgerührt, abfiltriert und getrocknet.
Es wurde zweimal aus 1,4-Dioxan umkristallisiert. Nach Trocknung im Vakuum bei 120°C erhielt man 229 g (390.5 mmol, 67%) 2,7-Dibrom-2',7'-di-tert-butylspirobifluoren als farblosen Feststoff, welcher lt. HPLC (Zorbax SB-C18 3.5µm, 4.6 x 75mm, 85% Methanol/10% THF/5% Wasser, 1ml/min, UV Detektion 290-320 nm) eine Reinheit von >99.8% aufwies.
¹H NMR (CDCl₃, 400 MHz): 7.70 (dd, 2 H, H4/5, J = 8.1, 0.6 Hz); 7.67 (d, 2 H, H4'/5', J = 8.1 Hz); 7.48 (dd, 2H, H3/H6, J = 8.3, 1.8 Hz); 7.41 (dd, 2H, H3'/H6', J = 8.1, 1.6 Hz); 6.83 (d, 2H, H1/8, J = 1.6 Hz); 6.62 (d, 2H, H1'/8', J = 1.3 Hz); 1.18 (s, 18 H, t-Butyl).

### Herstellung von 2,7-Di-t-butyl-2',7'-di(trimethylsilyl)spirobifluoren

50 g (85.2 mmol) 2,7-Dibrom-2',7'-di-t-butylspirobifluoren wurden in 450ml destilliertem THF gelöst und auf -78°C gekühlt. 85.2 ml (213 mmol) einer 2.5M Lösung von Butyllithium in Hexan wurde langsam zugetropft. Man ließ eine Stunde bei dieser Temperatur rühren; dann wurde eine Lösung von 23.14 g (213 mmol, 27 ml) Chlortrimethylsilan in 50 ml destilliertem THF zugetropft und über Nacht auf Raumtemperatur erwärmt. Die Reaktionslösung wurde auf 400 ml Eiswasser und 7 ml konzentrierte Salzsäure gegossen, die wäßrige Phase wurde noch mit 30 ml Essigester extrahiert. Die vereinigten org. Phasen wurden mit 100 ml Essigester versetzt und 3x mit je 100 ml NaHCO₃ Lösung gewaschen. Es wurde mit MgSO₄ getrocknet, vom Lösungsmittel befreit und der Rückstand (61.12g) wurde aus Essigester umkristallisiert. Man erhielt 41.82 g (73.0 mmol, 86%) 2,7-Di-t-butyl-2',7'bis(trimethylsilyl)spirobifluoren als farblosen Feststoff, der lt.¹H NMR > 99%ig war.
¹H NMR (CDCl₃, 400 MHz): 7.83 (dd, 2 H, H4/5, J = 8.1, 0.7 Hz); 7.72 (d, 2 H, H4'/5', J = 8.0 Hz); 7.51 (dd, 2H, H3/H6, J = 7.5, 1.3 Hz); 7.37 (dd, 2H, H3'/H6', J = 8.0, 1.6 Hz); 6.79 (d, 2H, H1/8, J = 1.6 Hz); 6.72 (d, 2H, H1'/8', J = 1.3 Hz); 1.13 (s, 18 H, t-Butyl); 0.09 (s, 18 H, CH₃-Si).

### Herstellung von 2',7'-Di-t-butyl-spirobifluoren-2,7-bisboronsäureglycolester (M4)

30.0 g (52.3 mmol) 2,7-Di-t-butyl-2',7'-bis(trimethylsilyl)spirobifluoren wurden unter N₂ in 100 ml trockenem Methylenchlorid gelöst, auf-78°C gekühlt und mit 39.3 g (157 mmol, 3 eq, 14.8 ml) Bortribromid versetzt. Es wurde über Nacht auf Raumtemperatur erwärmt, mit weiteren 100 ml Methylenchlorid versetzt und auf 500 ml Wasser/40 g NaOH gegossen. Der ausgefallene weiße Niederschlag wurde im Vakuum getrocknet. Der Niederschlag wurde in 150 ml Chloroform gelöst, mit 10.1 ml (162.9 mmol) Ethylenglycol sowie 0.2 ml konzentrierter H₂SO₄ versetzt und 5 Stunden auf Rückfluß erhitzt. Der beim Abkühlen entstandene Feststoff wurde abgesaugt und aus Chloroform umkristallisiert. Man erhielt 20.1 g (35.4 mmol, 67%) 2',7'-Di-t-butyl-spirobifluoren-2,7-bisboronsäureglycolester als farblosen Feststoff, welcher laut ¹H NMR eine Reinheit von >99% aufwies.
¹H NMR (CDCl₃, 400 MHz): 7.90 (dd, 2 H, H4/5, J = 8.1, 0.7 Hz); 7.84 (dd, 2 H, H4'/5', J = 7.5, 1.0 Hz); 7.66 (d, 2H, H3/H6, J = 8.0 Hz); 7.33 (dd, 2H, H3'/H6', J = 8.0, 2.0 Hz); 7.17 (br. s, 2H, H1/8); 6.57 (d, 2H, H1'/8', J = 1.6 Hz); 4.26 (s, 8H, Ethylenglycolester); 1.13 (s, 18 H, t-Butyl).

### A2: Monomere für Einheiten gemäß Formel (II) (Fluorene)

### Beispiel M5/M6: Darstellung von 2,7-Dibrom-9-(2,5-dimethylphenyl)-9-[4-(3,7-dimethyl-octyloxy)phenyl]fluoren und des entsprechenden Bisboronsäureesters

i) Herstellung von 2,7-Dibrom-9-(2,5-dimethylphenyl)fluoren-9-ol
ii) Herstellung von 2,7-Dibrom-9-(2,5-dimethylphenyl)-9-(4-hydroxyphenyl)fluoren
iii) Herstellung von 2,7-Dibrom-9-(2,5-dimethylphenyl)-9-[4-(3,7-dimethyloctyloxy)phenyl]fluoren **(M5)**
iv) Grignard Reaktion von 2,7-Dibrom-9-(2,5-dimethylphenyl)-9-[4-(3,7-dimethyloctyloxy)phenyl]fluoren zu 9-(4-(3,7-dimethyloctyloxy)phenyl)-9-(2,5-dimethylphenyl)fluoren-2,7-bisboronsäure-bisethylenglycolester **(M6)**
Die Herstellung dieser Monomere ist in WO 00/22026 beschrieben

### Beispiel M7/M8: Darstellung von 2,7-Dibrom-9-(2',5'-dimethylphenyl)-9 [3",4"-bis(2- methyl-butyloxy)phenyl]fluoren und des entsprechenden Bisboronsäureesters

### Herstellung von 2,7-Dibrom-9-(2',5'-dimethylphenyl)-9-(3",4"-bishydroxyphenyl)fluoren

99.17 g (900.6 mmol) Catechol und 200.0 g (450.3 mmol) 2,7-Dibrom-9-(2,5-dimethylphenyl)-9-hydroxy-fluoren wurden in 700ml Toluol gelöst. Die weiße Suspension wurde auf 60°C erwärmt. Nach Zugabe von 2.39 g (22.5 mmol, 1.96ml) 3-Mercaptopropionsäure wurden innerhalb von 25 Minuten langsam ohne weitere Wärmezufuhr 81.7 ml konz. H₂SO₄ (150.4 g, 1533 mmol) zugetropft. Die Suspension wurde bei 60-65° 2 Stunden nachgerührt. Der angefallene Feststoff wurde abgesaugt, in 500 ml Essigester gelöst und mit 1200ml gesättigter Na₂CO₃ Lösung ausgerührt. Die Phasen wurden getrennt und die organische Phase wurde mit gesättigter Na₂CO₃ Lösung ausgeschüttelt bis kein Catechol mehr vorhanden war. Danach wurde zweimal mit je 200ml Wasser ausgeschüttelt und anschließend einrotiert. Es wurde aus Hexan/Essigester umkristallisiert. Man erhielt 201.2 g (375.2 mmol, 83%) 2,7-Dibrom-9-(2',5'-dimethylphenyl)-9-(3",4"-bis-hydroxyphenyl)fluoren als farblosen Feststoff. Lt. ¹H NMR war die Reinheit >99%.
¹H NMR (DMSO-d6, 500 MHz): 8.87 (br. s, 2 H, 2 x OH); 7.90 (d, 2 H, H4/H5, J = 8.1 Hz); 7.59 (dd, 2 H, H3/H6, J = 8.0,1.6 Hz); 7.41 (d, 2 H, H1, H8, J = 1.5 Hz); 6.98-6.94 (m, 2H, H5', H6'); 6.89 (br. s, 1H, H6'); 6.64 (d, 1H, H3", J = 8.3 Hz); 6.56 (d, 1H, H6", J = 2.3 Hz); 6.43 (dd, 1H, H4", J = 8.3, 2.3 Hz); 2.16 (s, 3H, CH₃ an C5"); 1.42 (br. s, 3H, CH₃ an C2").

### Herstellung von 2,7-Dibrom-9-(2',5'-dimethylphenyl)-9-[3,"4"-bis(2-methylbutyloxy)phenyl]fluoren (M7)

76.1 g (550 mmol, 2.2 eq) gemahlenes Kaliumcarbonat wurden in 270 ml trockenen Dimethylformamid suspendiert. Man fügte portionsweise 134.1 g (250 mmol) 2,7-Dibrom-9-(2,5-dimethylphenyl)-9-(3,4-dihydroxyphenyl)-fluoren zu und tropfte innerhalb von 20 min 154.6 g (600 mmol, 2.4 eq) 1-Toluolsulfonyloxy-2-methylbutan zu. Die Mischung wurde 18 Stunden auf 85°C erhitzt. Es wurden noch weitere 25.5 g Kaliumcarbonat und 51.6 g (200 mmol) 1-Toluolsulfonyloxy-2-methylbutan zugeben und weitere 30 Stunden erhitzt. Die Reaktionsmischung wurde abgekühlt und das ausgefallene Produkt wurde abgesaugt und mit Hexan gewaschen. Der weiße Feststoff wurde 4 x mit je 200 ml Ethanol ausgerührt und getrocknet. Nach Umkristallisation aus 1,4-Dioxan erhielt man 169.3 g (197.6 mmol, 79%) 2,7-Dibrom-9-(2',5'-dimethylphenyl)-9-[3",4"-bis(2-methyl-butyloxy)phenyl]fluoren (M7) als farblosen Feststoff.
¹H NMR (CDCl₃, 500 MHz): 7.56 (2 H, H4/H5, J = 8.3 Hz); 7.46 (d, 2 H, H3/H6, J = 8.1 Hz); 7.55-7.35 (br. m, 2 H, H1/H8); 6.98 u. 6.93 (2 x d, je 1H, H3'/H4', J = 7.5 Hz), 6.94 (br. s, 1H, H6'); 6.84 (d, 1H, H2", J = 2.3); 6.65 (d, 1H, H5", J = 8.3 Hz); 6.52 (dd; 1H, H6", J = 8.3 2.3 Hz); 3.8-3.6 (m, 4H, OCH₂); 2.21 (s, 3H, CH₃ an C5'); 1.90-1.77 (m, 2H, H-C); 1.60-1.48 (m, 2H, CH₂); 1.47 (br. s, 3H, CH₃ an C2');1.30-1.18 (m, 2H, CH₂); 1.01-0.88 (m, 12 H, 4 x CH₃).

### Herstellung von 9-(3",4"-Bis(2-methylbutyloxy)phenyl)-9-(2',5'-dimethylphenyl)fluoren-2,7-bisboronsäure-bis-pinacoylester (M8)

92.0 g (136 mmol) 2,7-Dibrom-9-(3",4"-bis(2-methylbutyloxy)phenyl)-9-(2',5'dimethylphenyl)fluoren wurden in 250 ml THF gelöst. 6.96 g (286 mmol) Mg wurden vorgelegt und unter Zugabe einer Spatelspitze lod mit 10% der Menge der Eduktlösung versetzt. Nach Anspringen der Reaktion wurde die restliche Lösung innerhalb einer halben Stunde zugetropft und dann noch 3 Stunden unter Rückfluß erhitzt.
76.0 g (408 mmol, 83.4 ml) Isopropylpinacolylborat wurden in 200 ml THF gelöst und auf -70°C gekühlt. Die Grignardlösung wurde innerhalb von einer Stunde zugetropft; es wurde noch 3 h bei -70°C gerührt und dann über Nacht aufgetaut.
Es wurde dann bei Raumtemperatur erst 65.34 g (62.3 ml, 1088 mmol) Essigsäure in 250 ml Wasser, dann 200 ml Essigester zugesetzt. Die organische Phase wurde mit 100 ml Wasser gewaschen, mit MgSO₄ getrocknet und einrotiert. Man erhielt 89.2g Rohprodukt, welches aus n-Hexan / iso-Propanol umkristallisiert wurde. Es wurden 82.6g (107.2 mmol, 78 %) eines Feststoffes erhalten, welcher lt. ¹H NMR und HPLC (Zorbax SB-C18 3.5µm, 4.6 x 75mm, 98% Acetonitril/2% Pinacol, 1ml/min, UV Detektion 230-330 nm) eine Reinheit >99.8% aufwies.
¹H NMR (CDCl₃, 500 MHz): 7.92-7.63 (m, 6 H, H-Fluoren); 7.08 (d, 1 H, H2", J = 2.4); 7.05 (br. s, 1H, H6'), 6.95 u. 6.86 (2 x d, je 1H, H3'/H4', J = 7.7), 6.60-6.54 (m, 2H, H5", H6"); 3.8-3.6 (m, 4H, OCH₂); 2.23 (s, 3H, CH₃-C5'); 1.94-1.77 (m, 2H, C-H); 1.59-1.48 (m, 2H, CH₂); 1.31 (br. s, 3H, CH₃ an C2');1.28-1.18 (m, 2H, CH₂); 1.00-0.85 (m, 12 H, 4 x CH₃).

### Beispiel M14/M15: Darstellung von 2,7-Dibrom-9,9-bis-(2-ethylhexyl)fluoren und des entsprechenden Bisboronsäureesters

Die Herstellung von 2,7-Dibrom-9,9-bis-(2-ethylhexyl)fluoren **(M14)** und 9,9-Bis-(2-ethylhexyl)fluoren-2,7-bisboronsäure-bisglykolester **(M15)** ist in WO 00/22027 beschrieben.

### A3: Monomere für Einheiten gemäß Formel (III) bis (V) (Triarylamine, Phenylendiaminderivate und Tetraarylbenzidine)

### Herstellung von N,N'-Diphenyl-N,N'-bis(4-tert-butylphenyl)benzidin

Unter N₂ und Lichtausschluß wurden 30.79 g (91.53 mmol) N,N'-Diphenylbenzidin und 42.92 g (201.4 mmol) 1-Brom-4-tert-butylbenzol in 600 ml destilliertem Toluol gelöst. Man fügte dann 740.4 mg (3.66 mmol) Tris-o-tolylphosphin, 412.2 mg (1.83 mmol) Palladiumacetat und 22.75 g (236.5 mmol) NaOtBu zu. Die Suspension wurde für 1 Stunde auf 90°C erhitzt.
Nach Abkühlen auf Raumtemperatur wurde von angefallenem Niederschlag abgesaugt und die Mutterlauge wurde mit 400 ml 1%iger NaCN-Lösung ausgerührt. Die wäßrige Phase wurde noch zweimal mit je 200 ml Essigester extrahiert, die vereinigten organischen Phasen wurden zweimal mit je 100 ml Wasser gewaschen. Es wurde über Na₂SO₄ getrocknet und das Lösungsmittel wurde mit einem Rotationsverdampfer entfernt. Der erhaltene Feststoff wurde mit Hexan ausgerührt und aus Essigester umkristallisiert. Man erhielt 28.98 g (48.2 mmol, 53%) N,N'-Diphenyl-N,N'-bis(4-tert-butyl)phenylbenzidin als farblosen Feststoff, welcher laut HPLC (Zorbax SB-C18 3.5µm, 4.6 x 75mm, 85% Methanol/10% THF/5% Wasser, 1ml/min, UV Detektion 280-380 nm) eine Reinheit >99.9% aufwies.
¹H NMR (CDCl₃ + N₂H₄·H₂O, 500 MHz): 7.43 (d mit FS, 4H, H-Arom, J = 8.7 Hz); 7.32-7.21 (m, 8H, H-Arom); 7.13-7.08 (m, 8H, H-Arom); 7.04 (d mit FS, 4H, H-Arom, J = 8.6 Hz); 7.01-6.96 (m, 2H, H-Arom); 1.32 (s, 18H, t-Butyl).

### Herstellung von N,N'-Diphenyl-N, N'-bis(4-methoxyphenyl)benzidin

Analog dem vorangegangenen Beispiel wurden 10 g (29.72 mmol) N,N'-Diphenylbenzidin, 12.23 g (65.4 mmol) 1-Brom-4-methoxybenzol mit 362 mg (1.19 mmol) Tris-o-tolylphosphin, 7.43 g (77.4 mmol) NaO*t*Bu und 132mg (0.58 mmol) Palladiumacetat in 200 ml destilliertem Toluol umgesetzt. Nach analoger Aufarbeitung und Umkristallisation aus Hexan erhielt man 12.7 g (73%) eines Feststoffes, welcher laut HPLC (Zorbax SB-C18 3.5µm, 4.6 x 75mm, 85% Methanol/10% THF/5% Wasser, 1ml/min, UV Detektion 280-380 nm) eine Reinheit >99.8% aufwies.
¹H NMR (CDCl₃ + N₂H₄·H₂O, 500 MHz): 7.41 (d mit FS, 4H, H-Arom, J = 8.7 Hz); 7.25 - 7.20 (m, 4H, H-Arom); 7.12 - 7.05 (m, 12H, H-Arom); 6.98-6.92 (m, 2H, H-Arom); 6.85 (d mit FS, 4H, H-Arom, J = 8.6 Hz); 3.81 (s, 6H, OCH₃).

### Herstellung von N,N'-Bis(4-bromphenyl)-N,N'-bis(4-tert-butylphenyl)benzidin (M9)

10.57g (17.59 mmol) N,N'-Diphenyl-N,N-bis(4-tert-butylphenyl)benzidin wurden in 100 ml Chloroform gelöst, mit einem Tropfen Hydrazinhydrat versetzt und auf 0°C gekühlt. 6.26 g (35.18 mmol, 2 eq) N-Bromsuccinimid wurden nun innerhalb von 15 Minuten portionsweise zugegeben. Es wurde noch 20 Minuten bei dieser Temperatur gerührt. Der gebildete Niederschlag wurde abgesaugt und es wurde mit 100 ml gesättigter Na₂SO₃-Lösung gewaschen. Die wäßrige Phase wurde mit 100 ml Chloroform extrahiert und die vereinigten Organischen Phasen wurden zweimal mit je 100 ml Wasser gewaschen und mit MgSO₄ getrocknet. Nach Abziehen des Lösungsmittels und Umkristallisation aus Essigester erhielt man 10.45 g (13.8 mmol, 78%) N,N'-Bis(4-bromophenyl)-N,N'-bis(4-tert-butylphenyl)benzidin als farblosen Feststoff, welcher laut HPLC (Zorbax SB-C18 3.5µm, 4.6 x 75mm, 85% Methanol/10% THF/5% Wasser, 1ml/min, UV Detektion 280-380 nm) eine Reinheit >99.8 % aufwies.
¹H NMR (CDCl₃ + N₂H₄*H₂O, 500 MHz): 7.43 (d mit FS, 4H, H-Arom, J = 8.7 Hz); 7.32 (d mit FS, 4H, H-Arom, J = 8.7 Hz); 7.28 (d mit FS, 4H, H-Arom, J = 8.7 Hz); 7.09 (d mit FS, 4H, H-Arom, J = 8.7 Hz); 7.03 (d mit FS, 4H, H-Arom, J = 8.7 Hz); 6.97 (d mit FS, 4H, H-Arom, J = 8.7 Hz); 1.32 (s, 18H, t-Butyl).

### Herstellung von N,N'-Bis(4-bromphenyl)-N,N'-bis(4-methoxyphenyl)benzidin(M10)

Analog der vorhergehenden Vorschrift wurden 12.6 g (22.96 mmol) N,N'-Diphenyl-N,N'-bis(4-methoxyphenyl)benzidin in 350 ml Chloroform mit 10.62 g (59.7 mmol, 2 eq) N-Bromsuccinimid umgesetzt und aus Essigester umkristallisiert. Man erhielt 9.81 g (13.9 mmol, 60%) N,N'-Bis(4-bromophenyl)-N,N'-bis(4-methoxyphenyl)benzidin als farblosen Feststoff M10, welcher laut HPLC (Zorbax SB-C18 3.5µm, 4.6 x 75mm, 85% Methanol/10% THF/5% Wasser, 1ml/min, UV Detektion 280-380 nm) eine Reinheit >99.5 % aufwies.
¹H NMR (CDCl₃ + N₂H₄·H₂O, 500 MHz): 7.40 (d mit FS, 4H, H-Arom, J = 9.0 Hz); 7.30 (d mit FS, 4H, H-Arom, J = 9.0 Hz); 7.07 und 7.05 (2 x d mit FS, je 4H, H-Arom, J ~ 8 Hz); 6.92 (d mit FS, 4H, H-Arom. J = 8.7 Hz); 6.85 (d mit FS, 4H, H-Arom, J = 8.7 Hz); 3.80 (s, 6 H, OCH₃).

### Herstellung von 4,4'-Dibromtriphenylamin (M11)

Die Synthese dieser Verbindung ist in DE 19.981.010 beschrieben.

### A4: Monomere für Einheiten gemäß Formel (Vl) bis (XXXXV)

### Herstellung von 4,7-Dibrom-benzo[1,2,5]thiadiazol (M12)

Die Herstellung dieser Verbindung ist in J. Heterocycl. Chem. **1970,** 629-633 beschrieben.
Herstellung von 4,7-Dibromobenzofurazon (**M13**)
Die Herstellung dieser Verbindung ist in J. Chem. Soc. **1931,** 3308-3311 beschrieben.

Für die bessere Übersichtlichkeit sind die Monomere, deren Synthese hier aufgeführt wurde, in der folgenden Grafik zusammengefaßt.

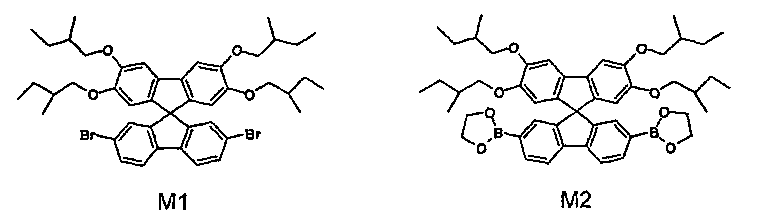

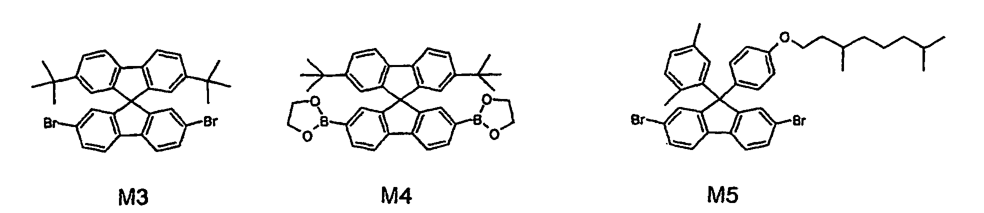

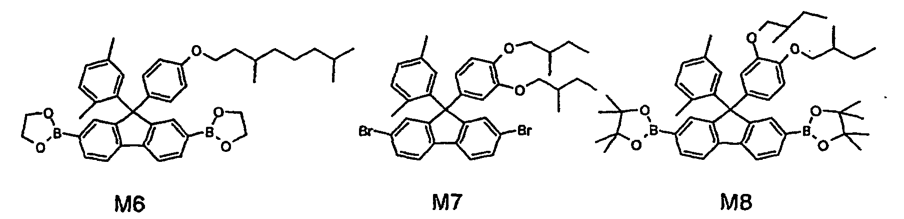

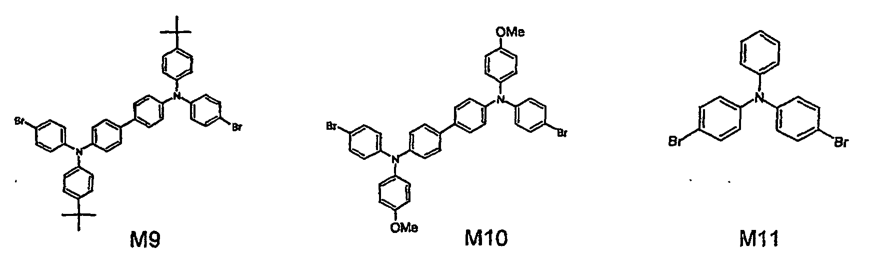

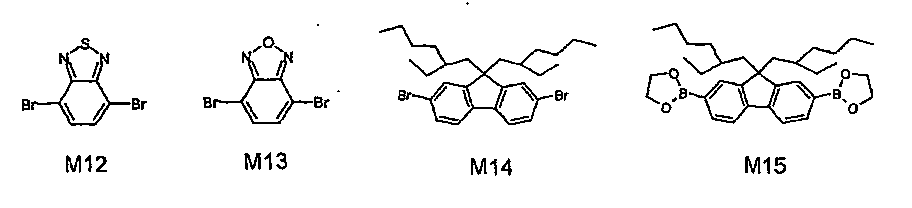

Weitere Monomere wurden in Analogie bzw. gemäß den oben zitierten Literaturstellen dargestellt. Diese sind in der nachfolgende Aufstellung zusammengefaßt:

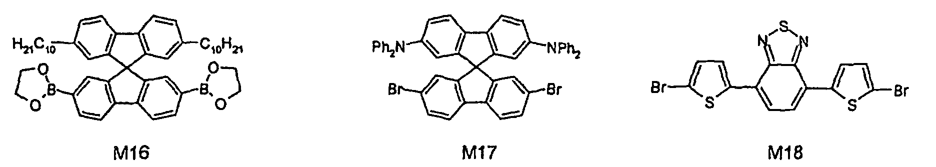

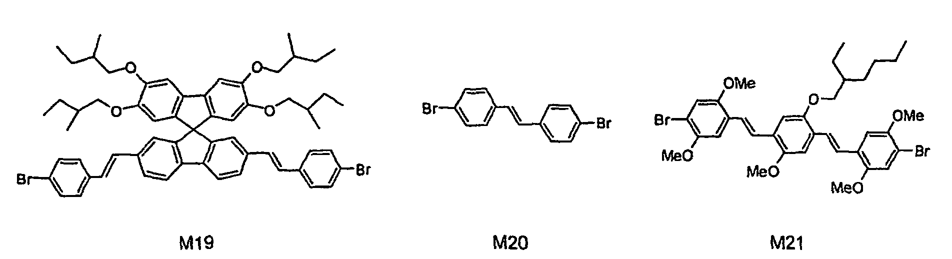

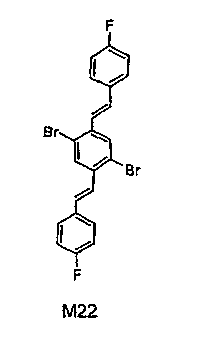

### Teil B: Herstellung der Polymere

### Beispiel P1: Copolymerisation von 50 mol% 2,7-Dibromo-2,3',6',7'-tetra(2-methylbutyloxy)spirobifluoren (M1), 40 mol % 2,7-Dibrom-9-(2',5'-dimethyl-phenyl)-9-[3",4"-bis(2-methyl-butyloxy)phenyl]fluoren (M7) und 10 mol% von N,N'-Bis(4-bromo)phenyl-N,N'-bis(4-tert-butyl)phenylbenzidin (M9) durch Yamamoto-Kupplung (Polymer P1)

Unter Argon wurden 25 ml Dimethylformamid und 80 ml Toluol auf 80° C erwärmt und 1.53 g (5.57 mmol) Ni(COD)₂ sowie 0.87 g (5.57 mmol) 2,2'-Bipyridyl wurden zugegeben. Nach 30 Minuten wurde zuerst 0.379 g (3.51 mmol, 0.43 ml) 1,5-Cyclooctadien, dann eine Lösung von 0.990 g (1.21 mmol) 2,7-Dibromo-2',3',6',7'tetra(2-methylbutyloxy)spirobifluoren, 0.652 g (0.968 mmol) 2,7-Dibrom-9-(2',5'dimethyl-phenyl)-9-[3",4"-bis(2-methyl-butyloxy)phenyl]fluoren und 0.183 g (0.242 mmol) N,N'-Bis(4-bromo)phenyl-N,N'-bis(4-tert-butyl)phenylbenzidin in 20 ml Toluol zugeben. Nach 144 h wurde abgekühlt, 5 ml HCI in Dioxan zugeben und die Reaktionsmischung wurde 15 min gerührt. Es wurde 50 ml Chloroform zugeben und 15 min gerührt. Die organische Phase wurde mit zweimal mit je100 ml 5M HCl und einmal mit 100 ml ges. NaHCO₃ Lösung gewaschen Die Lösung wurde in 450 ml Methanol gefällt und das rohe Polymer wurde abgesaugt. Es wurde noch zweimal aus jeweils 100 ml THF/150 ml Methanol umgefällt. Man erhielt 0.90 g (63%) faseriges, leicht gelbes Polymer P1.
¹H NMR (CDCl₃): 7.8-7.7 (m, 1 H, Spiro); 7.7-7.1 (m, 10.7 H, Fluoren, Spiro, TAD); 6.6 (br. s, 0.8H, Fluoren), 6.21 (m, 1H, Spiro); 4.0-3.4 (3 x m, 5.6 H, OCH₂), 2.16 (s, 1.2 H, CH₃); 1.9-0.7 (m, Alkyl H, darunter bei 1.30 t-Butyl).
GPC: THF; 1 ml/min, Plgel 10µm Mixed-B 2 x 300 x 7.5 mm², 35°C, Rl Detektion: Mw = 180000 g/mol, Mn = 79000 g/mol

### Beispiel P2 Copolymerisation von 2',3',6',7'-Tetra(2-methylbutyloxy)spirobifluoren-2,7-bisboronsäureethylenglycolester (M2), 2,7-Dibrom-9-(2',5'-dimethyl-phenyl)-9-[3",4"-bis(2-methyl-butyloxy)phenyl]fluoren (M7) und 92.5 mol% N,N'-Bis(4-bromophenyl)-N,N'-bis(4-tert-butylphenyl)benzidin (M9) durch Suzuki-Reaktion (Polymer P2).

5.0740 g (7.500 mmol) 2,7-Dibrorn-9-(2',5'-dimethyl-phenyl)-9-[3",4"-bis(2-methylbutyloxy)phenyl]fluoren, 8.0065g (10.00 mmol) 2',3',6',7'-Tetra(2-methylbutyloxy)spirobifluoren-2,7-bisboronsäureethylenglycolester, 1.8966 g (2.500 mmol) N,N'-Bis(4-bromophenyl)-N,N'-bis(4-tert-butylphenyl)benzidin, 9.67 g (42 mmol) K₃PO₄·H₂O, 30 ml Toluol, 15 ml Wasser und 0.25 ml Ethanol wurden 30 min durch Durchleiten von N₂ entgast. Anschließend wurde 175 mg (0.15 mmol) Pd(PPh₃)₄ unter Schutzgas zugegeben. Die Suspension wurde unter N₂-Überlagerung bei 87°C Innentemperatur (leichter Rückfluß) kräftig gerührt. Nach 4 Tagen wurden weitere 0.30 g 2',3',6',7'-Tetra(2-methylbutyloxy)spirobifluoren-2,7-bisboronsäureethylenglycolester zugesetzt. Nach weiteren 6 Stunden Erhitzen wurden 0.3 ml Brombenzol zugesetzt und noch 3 h zum Rückfluß erhitzt.
Die Reaktionslösung wurde mit 200 ml Toluol verdünnt, die Lösung wurde mit 200 ml 2%iger wäßriger NaCN 3h ausgerührt. Dabei hellte sich die Mischung nahezu vollständig auf. Die organische Phase wurde mit H₂O gewaschen und durch Zusetzen in 800 ml Ethanol gefällt. Das Polymer wurde in 200 ml THF 1h bei 40°C gelöst, mit 250 ml MeOH ausgefällt, gewaschen und im Vakuum getrocknet. In 200 ml THF/ 250 ml Methanol wurde ein weiteres Mal umgefällt, abgesaugt und bis zur Massenkonstanz getrocknet. Man erhielt 10.03 g (16.7 mmol, 84 %) des Polymeren P2 als leicht gelben Feststoff.
¹H NMR (CDCl₃): 7.8-7.7 (m, 1 H, Spiro); 7.7-7.1 (m, 10.75 H, Fluoren, Spiro, TAD); 6.6 (br. s, 0.75H, Fluoren), 6.21 (m, 1H, Spiro); 4.0-3.4 (3 x m, 5.5 H, OCH₂), 2.16 (s, 1.125 H, CH₃); 1.9-0.7 (m, Alkyl H, darunter bei 1.30 t-Butyl).
GPC: THF; 1 ml/min, PLgel 10µm Mixed-B 2 x 300 x 7.5 mm², 35°C, RI Detektion: Mw = 67000 g/mol, Mn = 29000 g/mol.

Weitere Polymere wurden analoge den Beschreibungen für P1 und P2 dargestellt. Die chemischen Eigenschaften sind in der folgenden Tabelle zusammengefaßt. Es wurden auch einige Vergleichspolymere (die jeweils entweder nur Einheiten gemäß Formel (I) bzw. solche gemäß Formel (11), aber eventuell auch noch weitere, enthalten) dargestellt. Auch diese sind in der Tabelle mit aufgeführt. All diese Polymere wurden auch für einen Einsatz in PLEDs untersucht. Wie PLEDs dargestellt werden können, ist zum einen oben schon ausgeführt und wird detaillierter noch in Teil C beschrieben.
Auch die wichtigsten Device-Eigenschaften (Farbe, Effizienz und Lebensdauer) sind in der Tabelle mit aufgeführt.

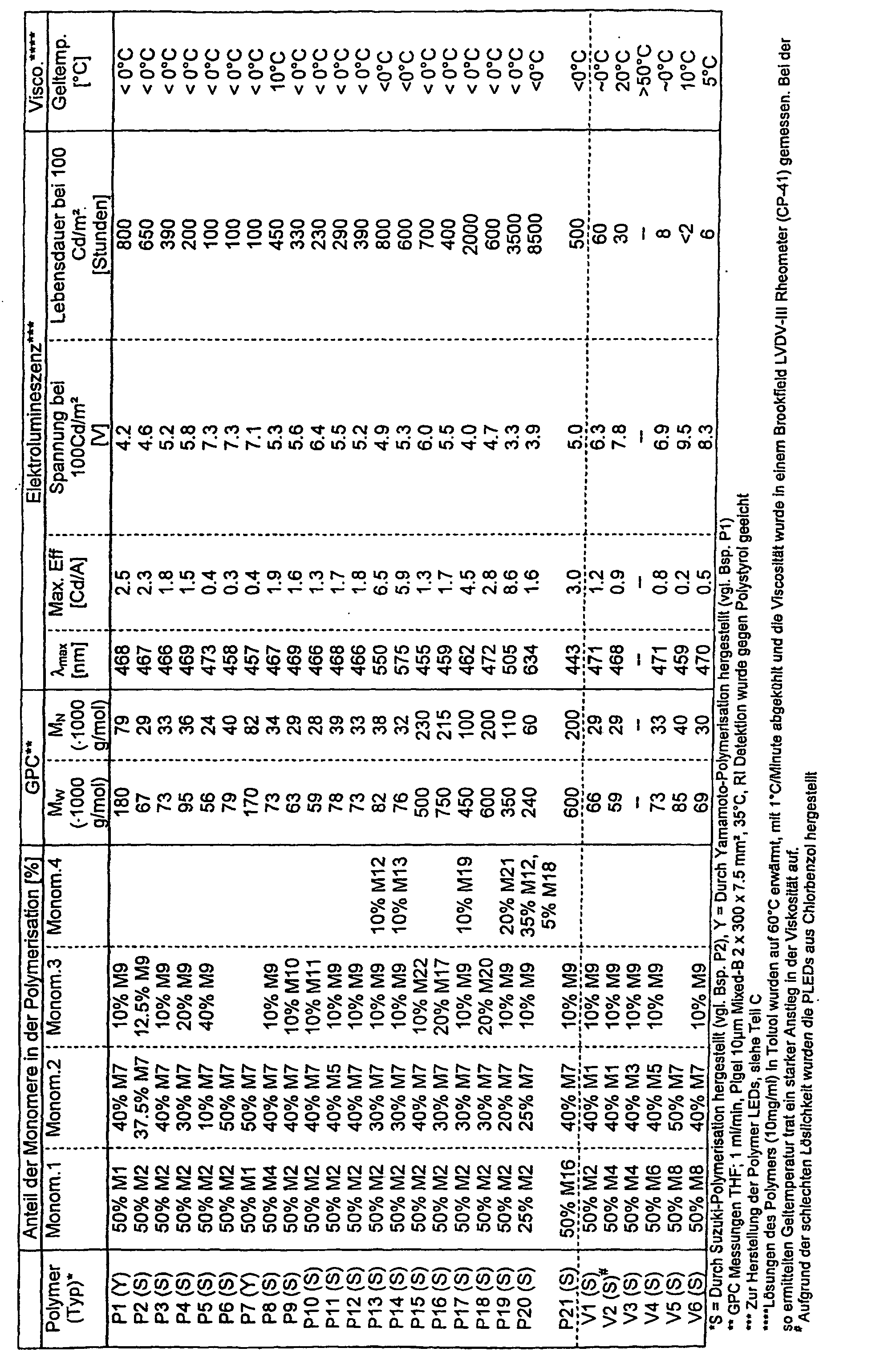

### Teil C: Herstellung und Charakterisierung von LEDs:

Die Herstellung von LEDs erfolgte nach dem im folgenden skizzierten allgemeinen Verfahren. Dieses mußte natürlich im Einzelfall auf die jeweiligen Gegebenheiten (z. B. Polymerviskosität und optimale Schichtdicke des Polymers im Device) angepaßt werden. Die im nachfolgenden beschriebenen LEDs waren jeweils Zweischichtsysteme, d. h. Substrat//ITO//PEDOT//Polymer//Kathode.
PEDOT ist ein Polythiophen-Derivat.

### Allgemeines Verfahren zur Herstellung von hocheffizienten, langlebigen LEDs:

Nachdem man die ITO-beschichteten Substrate (z. B. Glasträger, PET-Folie) auf die richtige Größe zugeschnitten hat, werden sie in mehreren Reinigungsschritten im Ultraschallbad gereinigt (z.B. Seifenlösung, Millipore-Wasser, Isopropanol). Zur Trocknung werden sie mit einer N₂-Pistole abgepustet und in einem Exsikkator gelagert. Vor der Beschichtung mit dem Polymer werden sie mit einem Ozon-Plasma-Gerät für ca. 20 Minuten behandelt. Von dem jeweiligen Polymer wird eine Lösung (in der Regel mit einer Konzentration von 4-25 mg/ml in beispielsweise Toluol, Chlorbenzol, Xylol:Cyclohexanon (4:1)) angesetzt und durch Rühren bei Raumtemperatur gelöst. Je nach Polymer kann es auch vorteilhaft sein, für einige Zeit bei 50 - 70°C zu rühren. Hat sich das Polymer vollständig gelöst, wird es durch einen 5µm Filter filtriert und bei variablen Geschwindigkeiten (400-6000) mit einem Spin-coater aufgeschleudert. Die Schichtdicken können dadurch im Bereich von ca. 50 und 300nm variiert werden. Vorab wird meist auf das (strukturierte) ITO ein leitfähiges Polymer, bevorzugt gedoptes PEDOT oder PANI aufgebracht. Auf die Polymerfilme werden noch Elektroden aufgebracht. Dies geschieht in der Regel durch thermisches Verdampfen (Balzer BA360 bzw. Pfeiffer PL S 500). Anschließend wird die durchsichtige ITO-Elektrode als Anode und die Metallelektrode (z. B. Ba, Yb, Ca) als Kathode kontaktiert und die Device-Parameter bestimmt.
Die mit den beschriebenen Polymeren erhaltenen Resultate sind in der Tabelle in Teil B zusammengefaßt. Für Polymer P1 ist in Abbildung 1 noch die typische IVL-Charakteristik in einer Test-Polymer-LED dargestellt.

## Patentansprüche

1. Konjugierte Polymere enthaltend wiederkehrende Einheiten der Formel (I) 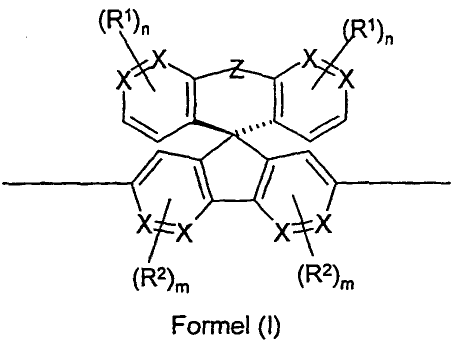 und wiederkehrende Einheiten der Formel (II) 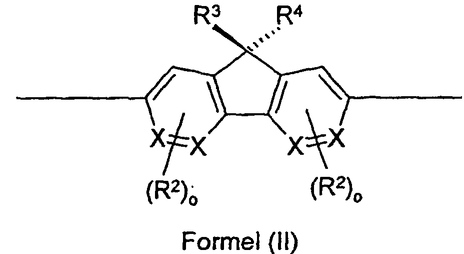 wobei der Anteil der Wiederholeinheiten vom Typ Formel (I) und Formel (II) zusammen mindestens 20% ausmachen und das Verhältnis der Wiederholeinheiten vom Typ Formel (I) zum Typ Formel (II) im Bereich von 1:10 bis 10:1 liegt, und die Symbole und Indizes die folgende Bedeutung haben:
X ist bei jedem Auftreten gleich oder verschieden C-H, CR¹ oder N,
Z ist bei jedem Auftreten gleich oder verschieden einer chemischen Einfachbindung, einer CR³R⁴-Gruppierung, einer -CR³R⁴-CR³R⁴-Gruppierung, einer -CR³=CR⁴-Gruppierung, O, S, N-R⁵, C=O, C=CR³R⁴ oder SiR³R⁴;
R¹ ist bei jedem Auftreten gleich oder verschieden eine geradkettige, verzweigte oder cyclische Alkyl- oder Alkoxykette mit 1 bis 22 C-Atomen, in der auch ein oder mehrere nicht benachbarte C-Atome durch N-R⁵, O, S, -CO-O-, O-CO-O ersetzt sein können, wobei auch ein oder mehrere H-Atome durch Fluor ersetzt sein können, eine Aryl- oder Aryloxygruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können, oder Cl, F, CN, N(R⁵)₂, wobei auch zwei oder mehrere Reste R¹ miteinander ein Ringsystem bilden können;
R² ist bei jedem Auftreten gleich oder verschieden eine geradkettige, verzweigte oder cyclische Alkyl- oder Alkoxykette mit 1 bis 22 C-Atomen, in der auch ein oder mehrere nicht benachbarte C-Atome durch N-R⁵, O, S, -CO-O-, O-CO-O ersetzt sein können, wobei auch ein oder mehrere H-Atome durch Fluor ersetzt sein können, eine Aryl- oder Aryloxygruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können, oder CN;
R³, R⁴ sind bei jedem Auftreten gleich oder verschieden H, eine geradkettige, verzweigte oder cyclische Alkylkette mit 1 bis 22 C-Atomen, in der auch ein oder mehrere nicht benachbarte C-Atome durch N-R⁵, O, S, -CO-O-, O-CO-O ersetzt sein können, wobei auch ein oder mehrere H-Atome durch Fluor ersetzt sein können, eine Arylgruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können, oder CN; die beiden Reste R³ und R⁴ können zusammen auch einen Ring ausbilden, der allerdings nicht zu Strukturen gemäß Formel (I) führt;
R⁵ ist bei jedem Auftreten gleich oder verschieden H, eine geradkettige, verzweigte oder cyclische Alkylkette mit 1 bis 22 C-Atomen, in der auch ein oder mehrere nicht benachbarte C-Atome durch O, S, -CO-O-, O-CO-O ersetzt sein können, wobei auch ein oder mehrere H-Atome durch Fluor ersetzt sein können, eine Arylgruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können;
n ist bei jedem Auftreten gleich oder verschieden 0,1, 2, 3, oder 4;
m ist bei jedem Auftreten gleich oder verschieden 0, 1, 2, oder 3;
o ist bei jedem Auftreten gleich oder verschieden 0, 1, 2, oder 3,
und der Maßgabe, daß bei mindestens einer Einheit gemäß Formel (1) mindestens ein Index n und/oder m von 0 verschieden ist.

2. Polymere gemäß Anspruch 1 **dadurch gekennzeichnet, daß** für das Symbol X = C-H oder C-R¹ gilt.

3. Polymere gemäß Anspruch 1 und/oder 2 **dadurch gekennzeichnet, daß** das Symbol Z für eine chemische Einfachbindung steht.

4. Polymere gemäß einem oder mehreren der Ansprüche 1 bis 3 **dadurch gekennzeichnet, daß** gilt:
R¹ ist bei jedem Auftreten gleich oder verschieden eine geradkettige, verzweigte oder cyclische Alkyl- oder Alkoxykette mit 1 bis 10 C-Atomen, wobei auch ein oder mehrere H-Atome durch Fluor ersetzt sein können, eine Arylgruppe mit 6 bis 14 C-Atomen, welche auch durch ein oder mehrere nicht-aromatische Reste R¹ substituiert ist.

5. Polymere gemäß einem oder mehreren der Ansprüche 1 bis 4 **dadurch gekennzeichnet, daß** gilt:
R¹ ist bei jedem Auftreten gleich oder verschieden eine geradkettige oder verzweigte Alkyl- oder Alkoxykette mit 1 bis 8 C-Atomen, oder eine Arylgruppe mit 6 bis 10 C-Atomen, welche auch durch ein oder mehrere nicht-aromatische Reste R¹ substituiert ist;
n ist gleich oder verschieden 1 oder 2.

6. Polymere gemäß einem oder mehreren der Ansprüche 1 bis 5 **dadurch gekennzeichnet, daß** gilt:
R² ist bei jedem Auftreten gleich oder verschieden eine geradkettige oder verzweigte Alkyl- oder Alkoxykette mit 1 bis 10 C-Atomen, wobei auch ein oder mehrere H-Atome durch Fluor ersetzt sein können, eine Aryl- oder Aryloxygruppe mit 6 bis 14 C-Atomen, welche auch durch ein oder mehrere nicht-aromatische Reste R¹ substituiert sein können, oder CN;
o, m ist bei jedem Auftreten gleich oder verschieden 0 oder 1.

7. Polymere gemäß einem oder mehreren der Ansprüche 1 bis 6 **dadurch gekennzeichnet, daß** gilt:
R² ist bei jedem Auftreten gleich oder verschieden eine geradkettige oder verzweigte Alkyl- oder Alkoxykette mit 1 bis 8 C-Atomen, wobei auch ein oder mehrere H-Atome durch Fluor ersetzt sein können, eine Arylgruppe mit 6 bis 10 C-Atomen, welche auch durch ein oder mehrere nicht-aromatische Reste R¹ substituiert sein können;
o, m ist bei jedem Auftreten gleich oder verschieden 0 oder 1, wobei für mindestens 50% aller im Polymer vorhandenen Wiederholeinheiten gemäß Formel (I) und (II) gilt, daß o und m gleich 0 ist.

8. Polymere gemäß einem oder mehreren der Ansprüche 1 bis 7 **dadurch gekennzeichnet, daß** gilt:
R³, R⁴ sind bei jedem Auftreten gleich oder verschieden eine geradkettige, verzweigte oder cyclische Alkylkette mit 1 bis 10 C-Atomen, in der auch ein oder mehrere nicht benachbarte C-Atome durch O ersetzt sein können, wobei auch ein oder mehrere H-Atome durch Fluor ersetzt sein können, eine Arylgruppe mit 5 bis 40 C-Atomen, bei der auch ein oder mehrere C-Atome durch O, S oder N ersetzt sein können, welche auch durch ein oder mehrere nicht-aromatische Reste R¹ substituiert sein können.

9. Polymere gemäß einem oder mehreren der Ansprüche 1 bis 8 **dadurch gekennzeichnet, daß** gilt:
R³, R⁴ sind bei jedem Auftreten gleich oder verschieden eine Arylgruppe mit 6 bis 14 C-Atomen, welche auch durch ein oder mehrere nicht-aromatische Reste R¹ substituiert sein können.

10. Polymere gemäß einem oder mehreren der Ansprüche 1 bis 9 **dadurch gekennzeichnet, daß** gilt:
R³, R⁴ sind bei jedem Auftreten gleich oder verschieden eine Arylgruppe mit 6 bis 14 C-Atomen, welche auch durch ein oder mehrere nicht-aromatische Reste R¹ substituiert sein können und bei denen jeweils an einer Einheit gemäß Formel (II) die Substituenten R³ und R⁴ voneinander unterschiedlich sind.

11. Polymere gemäß einem oder mehreren der Ansprüche 1 bis 10 **dadurch gekennzeichnet, daß** noch weitere Wiederholeinheiten enthalten sind, die andere Strukturen als diejenigen gemäß Formel (I) oder (II) aufweisen.

12. Polymere gemäß Anspruch 11 **dadurch gekennzeichnet, daß** mindestens ein weiteres Strukturelement Ladungstransporteigenschaften aufweist.

13. Polymere gemäß Anspruch 11 und/oder 12 **dadurch gekennzeichnet, daß** mindestens ein weiteres Strukturelement Lochtransporteigenschaften aufweist.

14. Polymere gemäß einem oder mehreren der Ansprüche 11 bis 13 **dadurch gekennzeichnet, daß** mindestens eines der folgenden Strukturelemente Triarylaminderivate, Benzidinderivate, Tetraarylen-para-phenylendiaminderivate, Phenothiazinderivate, Phenoxazinderivate, Dihydrophenazinderivate, Thianthrenderivate, Benzo-p-dioxinderivate, Phenoxathiinderivate, Carbazolderivate, Azulenderivate, Thiophenderivate, Pyrrolderivate oder Furanderivate enthalten ist.

15. Polymere gemäß einem oder mehreren der Ansprüche 11 bis 14 **dadurch gekennzeichnet, daß** mindestens 1% und höchstens 70% eines oder mehrerer der Struktrureinheiten gemäß den Formeln (III) bis (XIX) enthalten ist, 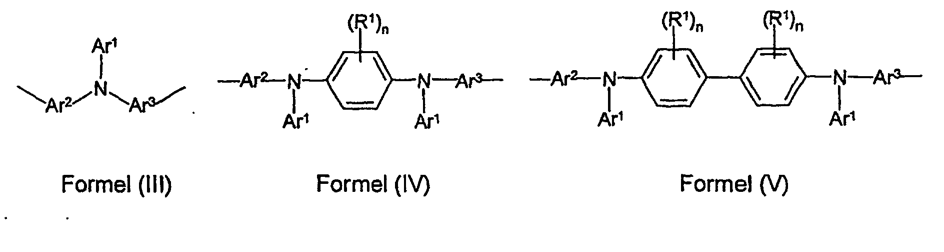 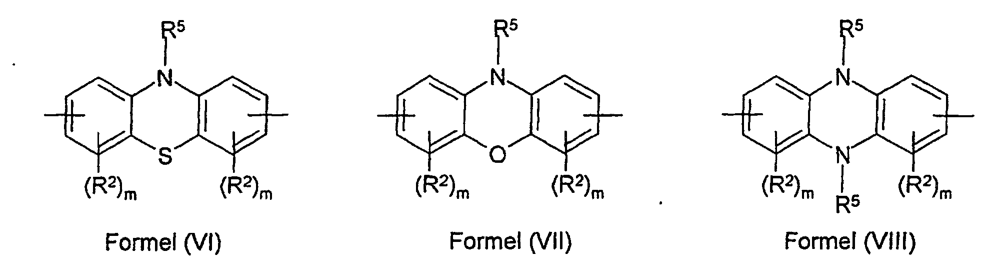 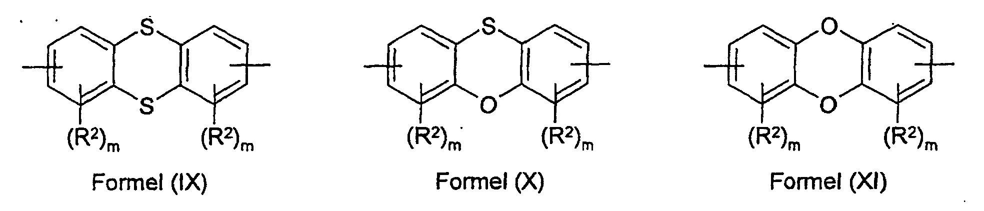 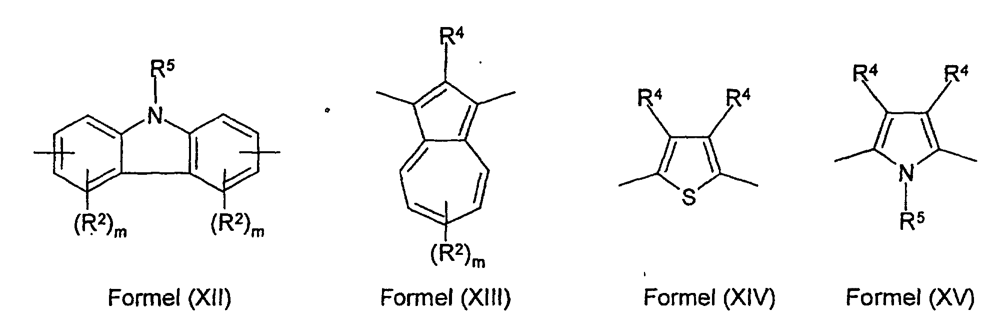 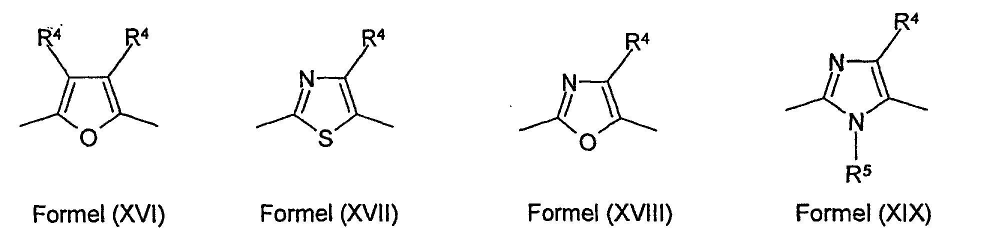 wobei die Symbole R¹ bis R⁵ und die Indizes n und m die in Anspruch 1 genannten Bedeutungen haben, und die weiteren Symbole
Ar¹, Ar², Ar³ bei jedem Auftreten gleich oder verschieden aromatischen oder heteroaromatischen Kohlenwasserstoffen mit 2 bis 40 C-Atomen, welche auch mit einem oder mehreren nicht-aromatischen Resten R¹ substituiert sein können,
bedeuten.

16. Polymere gemäß einem oder mehreren der Ansprüche 11 bis 15 **dadurch gekennzeichnet, daß** mindestens ein weiteres Strukturelement Elektronentransporteigenschaften aufweist.

17. Polymere gemäß Anspruch 16 **dadurch gekennzeichnet, daß** mindestens eines der folgenden Strukturelemente Pyridinderivate, Pyrimidinderivate, Pyridazinderivate, Pyrazinderivate, Oxadiazolderivate, Chinolinderivate, Chinoxalinderivate oder Phenazinderivate enthalten ist.

18. Polymere gemäß den Ansprüchen 16 und/oder 17 **dadurch gekennzeichnet, daß** mindestens 1% und höchstens 70% eines oder mehrerer der Struktrureinheiten gemäß den Formeln (XX) bis (XXX) enthalten ist, 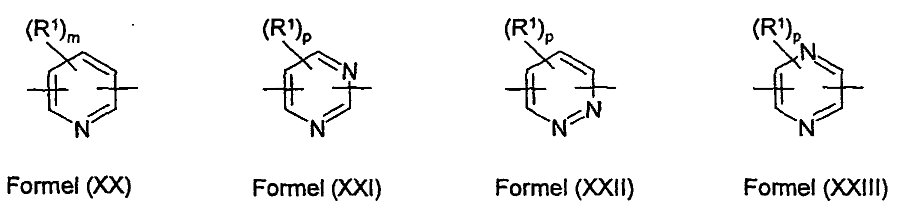 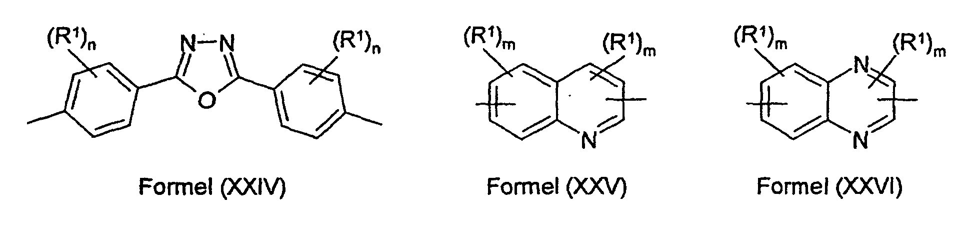 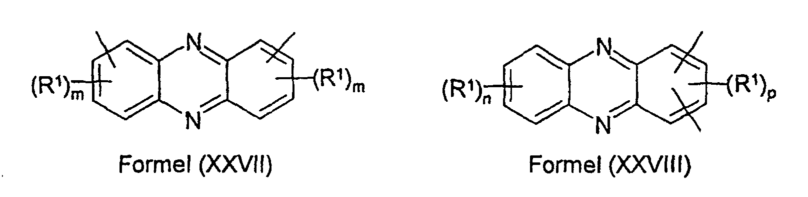 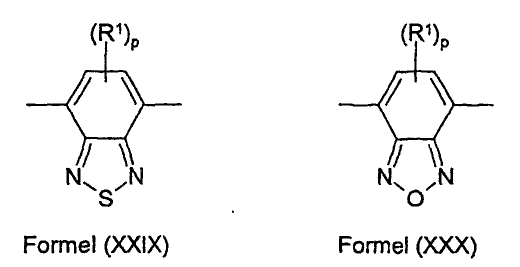 wobei das Symbol R¹ und die Indizes n und m die in Anspruch 1 genannten Bedeutungen haben, und der Index
p 0, 1 oder 2, bevorzugt 0 oder 1 bedeutet.

19. Polymere gemäß Anspruch 11 **dadurch gekennzeichnet, daß** sie mindestens ein weiteres Strukturelement mit Elektronentransporteigenschaften sowie mindestens ein weiteres Strukturelement mit Lochtransporteigenschaften enthalten.

20. Polymere gemäß Anspruch 19 **dadurch gekennzeichnet, daß** sie sowohl ein oder mehrere Struktureelemente gemäß den Formeln (III) bis (XIX) definiert in Anspruch 15, als auch ein oder mehrere Struktureelemente gemäß den Formeln (XX) bis (XXX) definiert in Anspruch 18, enthalten.

21. Polymere gemäß den Ansprüchen 19 und/oder 20 **dadurch gekennzeichnet, daß** das Polymer zusätzlich ein oder mehrere Struktrureinheiten gemäß den Formeln (XXXI) bis (XXXXV), 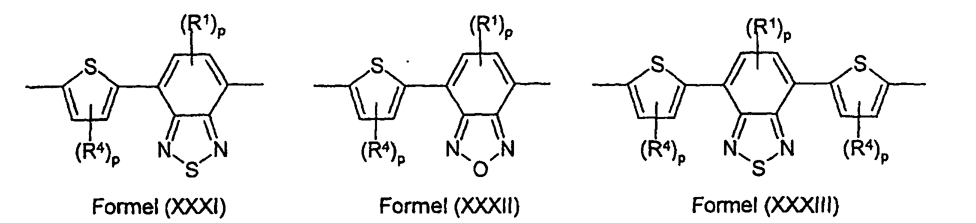 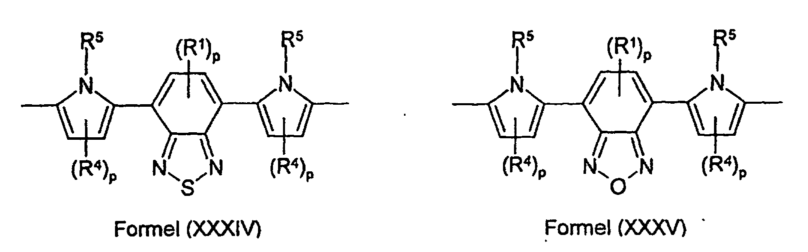 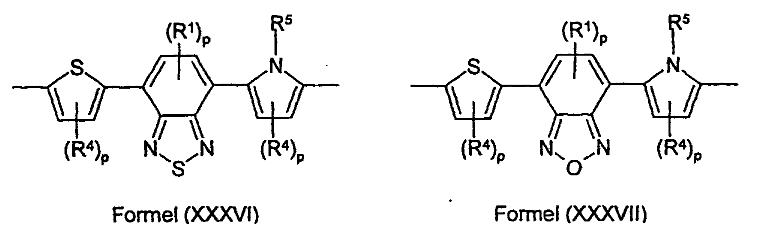 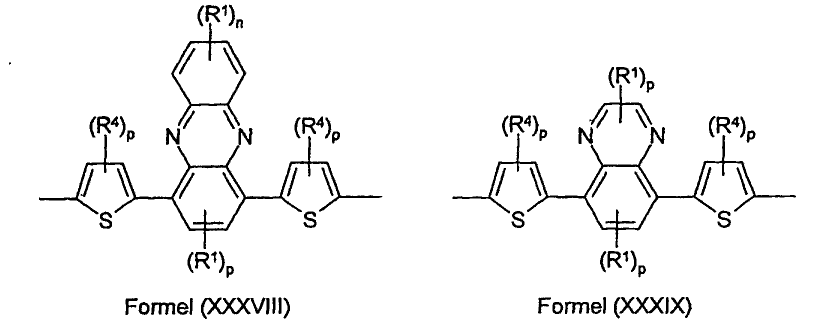 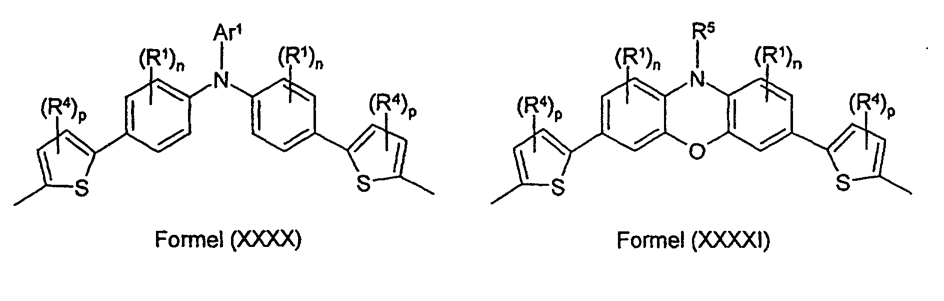 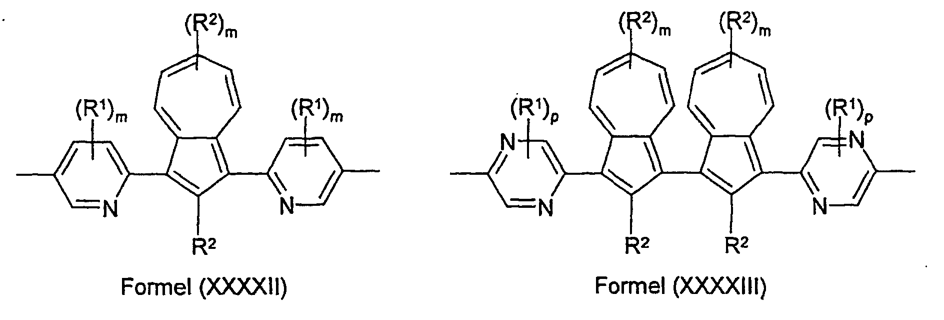 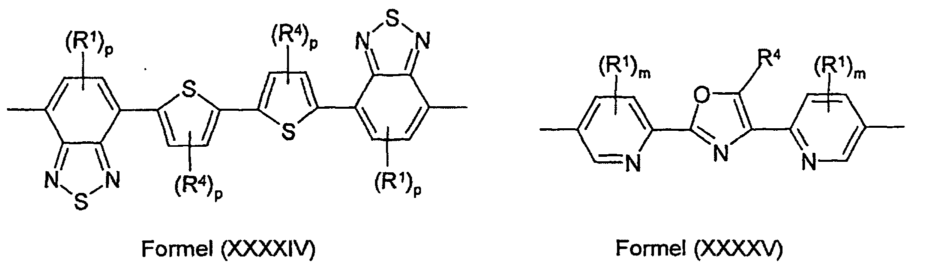 wobei die Symbole und Indizes die in den Ansprüchen 1 und 18 genannten Bedeutungen haben, enthalten ist.

22. Polymere gemäß einem oder mehreren der Ansprüche 11 bis 21, die mindestens noch eine weitere aromatische oder andere konjugierte Struktur aufweisen, welche die Ladungsträgermobilitäten nicht oder nur wenig beeinflussen.

23. Polymere gemäß Anspruch 22 **dadurch gekennzeichnet, daß** sie aromatischen Strukturen mit 6 bis 40 C-Atome oder Stilben- oder Bisstyrylarylenderivate aufweisen, welche mit einem oder mehreren nicht aromatischen Resten R¹ substituiert sein können.

24. Polymere gemäß den Ansprüchen 22 und/oder 23 **dadurch gekennzeichnet, daß** 1,4-Phenylen-, 1,4-Naphthylen-, 1,4- oder 9, 10-Anthracenylen-, 1,6- oder 2,7-oder 4,9-Pyren-, 3,9- oder 3,1 0-Perylen-, 2,7- oder 3,6-Phenanthren-, 4,4'-Biphenylen-, 4,4"-Terphenylen-, 4,4'-Bi-1,1'-naphthylen-, 4,4'-Stilben- oder 4,4"-Bisstyrylarylenderivate eingebaut sind.

25. Polymere gemäß einem oder mehreren der Ansprüche 11 bis 24 **dadurch gekennzeichnet, daß** metallorganische Komplexe in die Hauptkette inkorporiert sind.

26. Polymere gemäß Anspruch 25 **dadurch gekennzeichnet, daß** dabei d-Übergangsmetalle-Komplexe der höheren Metalle der Eisen-, Cobalt- und Nickeltriade, d. h. Komplexe von Ruthenium, Osmium, Rhodium, Iridium, Palladium und Platin enthalten sind.

27. Polymere gemäß den Ansprüchen 26 und/oder 27 **dadurch gekennzeichnet, daß** zusätzlich eine oder mehrere der Struktrureinheiten gemäß den Formeln (XXXXVI) bis (XXXXIX) enthalten ist, 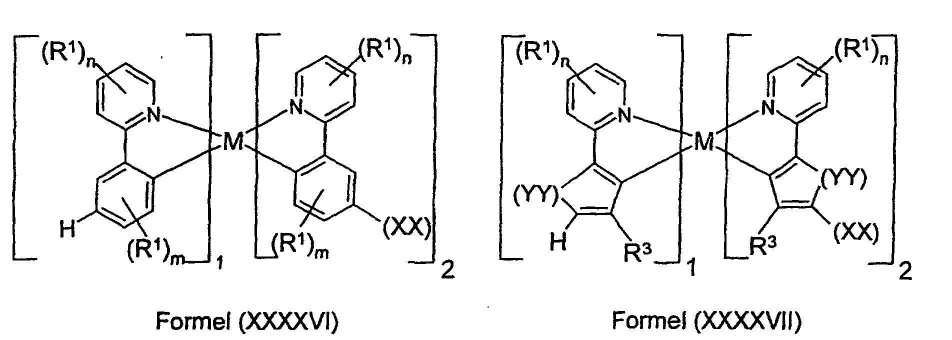 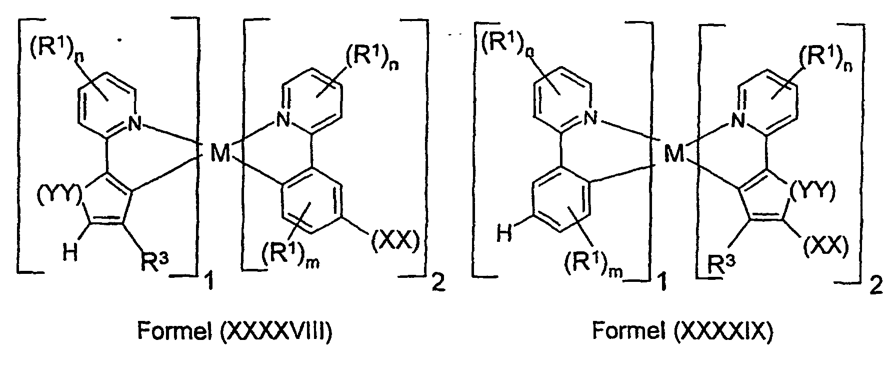 wobei die Symbol R¹ und R³ und die Indizes n und m die in Anspruch 1 genannten Bedeutungen haben, und
M Rh oder Ir,
XX der Verknüpfungsstelle im Polymer, und
YY bei jedem Auftreten gleich oder verschieden O, S oder Se
bedeutet.

28. Verwendung eines oder mehrerer der Polymere gemäß einem oder mehreren der Ansprüchen 1 bis 27 in einer PLED, insbesondere als Elektrolumineszenzmaterial.

29. PLED mit einer oder mehreren aktiven Schichten, wobei mindestens eine dieser aktiven Schichten ein oder mehrere erfindungsgemäße Polymere gemäß einem oder mehreren der Ansprüchen 1 bis 27 enthält.

30. Elektronisches Bauteil (Device) enthaltend ein oder mehrere erfindungsgemäße Polymere gemäß einem oder mehreren der Ansprüchen 1 bis 27.

31. Organische Integrierte Schaltungen (O-lCs), Organischen Feld-Effekt-Transistoren (OFETs), Organischen Dünnfilmtransistoren (OTFTs), Organische Solarzellen (O-SCs) oder Organische Laserdioden (O-Laser) **dadurch gekennzeichnet, daß** sie ein oder mehrere Polymere gemäß einem oder mehreren der Ansprüchen 1 bis 27 enthalten.

## Claims

1. A conjugated polymer comprising recurring units of the formula (I) 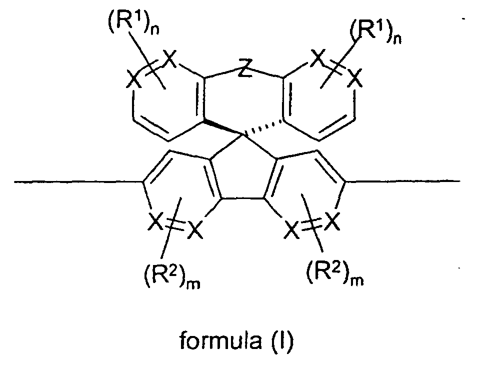 and recurring units of the formula (II), 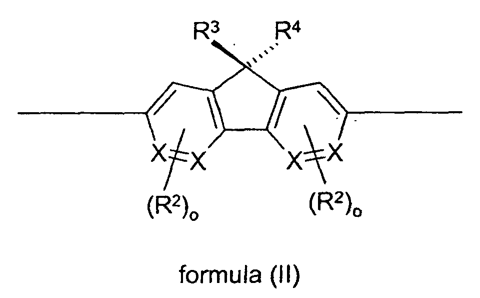 where the proportion of repeating units of the formula (I) and formula (II) together make up at least 20%, and the ratio of the repeating units of the formula (I) to those of the formula (II) is in the range from 1:10 to 10:1,
and the symbols and indices have the following meanings:
X is identical or different on each occurrence and is CH, CR1 or N,
Z is identical or different on each occurrence and is a single chemical bond, a CR3R4 group, a CR3R4-CR3R4 group, a CR3=CR4 group, O, S, N-R5, C=O, C=CR3R4 or SiR3R4;
R1 is identical or different on each occurrence and is a straight-chain, branched or cyclic alkyl or alkoxy chain which has from 1 to 22 carbon atoms and in which one or more nonadjacent carbon atoms may also be replaced by N-R5, O, S, -CO-O-, O-CO-O, where one or more H atoms may also be replaced by fluorine, an aryl or aryloxy group which has from 5 to 40 carbon atoms and in which one or more carbon atoms may also be replaced by O, S or N and which may also be substituted by one or more nonaromatic radicals R1, or Cl, F, CN, N(R5)2, where two or more radicals R1 may also be joined to form a ring system;
R2 is identical or different on each occurrence and is a straight-chain, branched or cyclic alkyl or alkoxy chain which has from 1 to 22 carbon atoms and in which one or more nonadjacent carbon atoms may also be replaced by N-R5, O, S, -CO-O-, O-CO-O, where one or more H atoms may also be replaced by fluorine, an aryl or aryloxy group which has from 5 to 40 carbon atoms and in which one or more carbon atoms may also be replaced by O, S or N and which may also be substituted by one or more nonaromatic radicals R1, or CN;
R3, R4 are identical or different on each occurrence and are each H, a straight-chain, branched or cyclic alkyl chain which has from 1 to 22 carbon atoms and in which one or more nonadjacent carbon atoms may also be replaced by N-R5, O, S, -CO-O-, O-CO-O, where one or more H atoms may also be replaced by fluorine, an aryl group which has from 5 to 40 carbon atoms and in which one or more carbon atoms may also be replaced by O, S or N and which may also be substituted by one or more nonaromatic radicals R1, or CN; the two radicals R3 and R4 may also be joined to form a ring which does not, however, lead to structures of the formula (I);
R5 is identical or different on each occurrence and is H, a straight-chain, branched or cyclic alkyl chain which has from 1 to 22 carbon atoms and in which one or more nonadjacent carbon atoms may also be replaced by O, S, -CO-O-, O-CO-O, where one or more H atoms may also be replaced by fluorine, an aryl group which has from 5 to 40 carbon atoms and in which one or more carbon atoms may also be replaced by O, S or N and which may also be substituted by one or more nonaromatic radicals R1;
n is identical or different on each occurrence and is 0, 1, 2, 3 or 4;
m is identical or different on each occurrence and is 0, 1, 2 or 3;
o is identical or different on each occurrence and is 0, 1, 2 or 3,
with the proviso that in the case of at least one unit of the formula (I), at least one index n and/or m is not 0.

2. A polymer as claimed in claim 1, **characterized in that** X = C-H or C-R1.

3. A polymer as claimed in claim 1 and/or 2, **characterized in that** the symbol Z represents a single chemical bond.

4. A polymer as claimed in one or more of claims 1 to 3, **characterized in that**:
R1 is identical or different on each occurrence and is a straight-chain, branched or cyclic alkyl or alkoxy chain which has from 1 to 10 carbon atoms and in which one or more H atoms may also be replaced by fluorine, or an aryl group which has from 6 to 14 carbon atoms and which is also substituted by one or more nonaromatic radicals R1.

5. A polymer as claimed in one or more of claims 1 to 4, **characterized in that**:
R¹ is identical or different on each occurrence and is a straight-chain or branched alkyl or alkoxy chain having from 1 to 8 carbon atoms or an aryl group which has from 6 to 10 carbon atoms and is also substituted by one or more nonaromatic radicals R1;
n are identical or different and are each 1 or 2.

6. A polymer as claimed in one or more of claims 1 to 5, **characterized in that**:
R2 is identical or different on each occurrence and is a straight-chain or branched alkyl or alkoxy chain which has from 1 to 10 carbon atoms and in which one or more H atoms may also be replaced by fluorine, an aryl or aryloxy group which has from 6 to 14 carbon atoms and which may also be substituted by one or more nonaromatic radicals R1, or CN;
o, m are identical or different on each occurrence and are each 0 or 1.

7. A polymer as claimed in one or more of claims 1 to 6, **characterized in that**:
R2 is identical or different on each occurrence and is a straight-chain or branched alkyl or alkoxy chain which has from 1 to 8 carbon atoms and in which one or more H atoms may also be replaced by fluorine, or an aryl group which has from 6 to 10 carbon atoms and which may also be substituted by one or more nonaromatic radicals R1;
o, m are identical or different on each occurrence and are each 0 or 1, with o and m being 0 for at least 50%, of all repeating units of the formulae (I) and (II) present in the polymer.

8. A polymer as claimed in one or more of claims 1 to 7, **characterized in that**:
R3, R4 are identical or different on each occurrence and are each a straight-chain, branched or cyclic alkyl chain which has from 1 to 10 carbon atoms and in which one or more nonadjacent carbon atoms may also be replaced by O, where one or more H atoms may also be replaced by fluorine, or an aryl group which has from 5 to 40 carbon atoms and in which one or more carbon atoms may also be replaced by O, S or N and which may also be substituted by one or more nonaromatic radicals R1.

9. A polymer as claimed in one or more of claims 1 to 8, **characterized in that**:
R3, R4 are identical or different on each occurrence and are each an aryl group which has from 6 to 14 carbon atoms and may also be substituted by one or more nonaromatic radicals R1.

10. A polymer as claimed in one or more of claims 1 to 9, **characterized in that**:
R3, R4 are identical or different on each occurrence and are each an aryl group which has from 6 to 14 carbon atoms and which may also be substituted by one or more nonaromatic radicals R1 and in which the substituents R3 and R4 are different from one another on a unit of the formula (II).

11. A polymer as claimed in one or more of claims 1 to 10, **characterized in that** further repeating units which have structures different from those of the formula (I) or (II) are additionally present.

12. A polymer as claimed in claim 11, **characterized in that** at least one further structural element has charge transport properties.

13. A polymer as claimed in claim 11 and/or 12, **characterized in that** at least one further structural element has hole transport properties.

14. A polymer as claimed in one or more of claims 11 to 13, **characterized in that** at least one of the following structural elements is present: triarylamine derivatives, benzidine derivatives, tetraarylene-para-phenylenediamine derivatives, phenothiazine derivatives, phenoxazine derivatives, dihydrophenazine derivatives, thianthrene derivatives, benzo-p-dioxin derivatives, phenoxathiine derivatives, carbazole derivatives, azulene derivatives, thiophene derivatives, pyrrole derivatives or furan derivatives.

15. A polymer as claimed in one or more of claims 11 to 14, **characterized in that** at least 1% and not more than 70% of one or more of the structural units of the formulae (III) to (XIX), 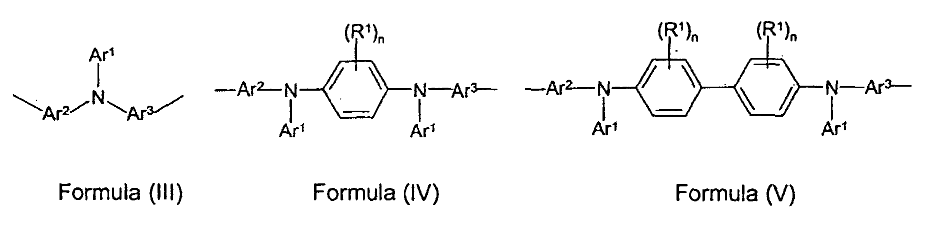 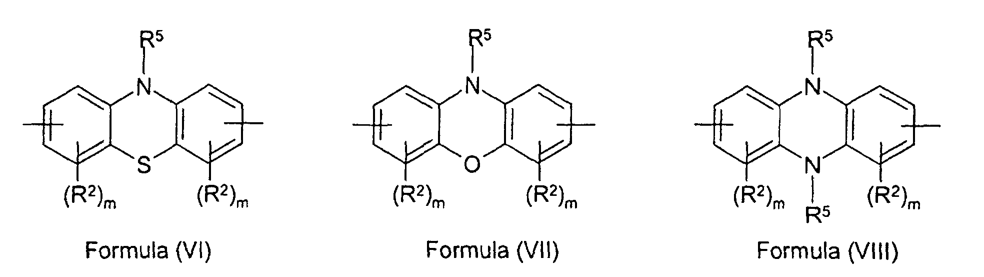 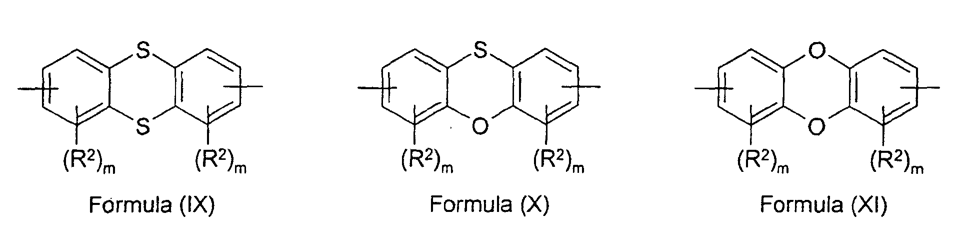 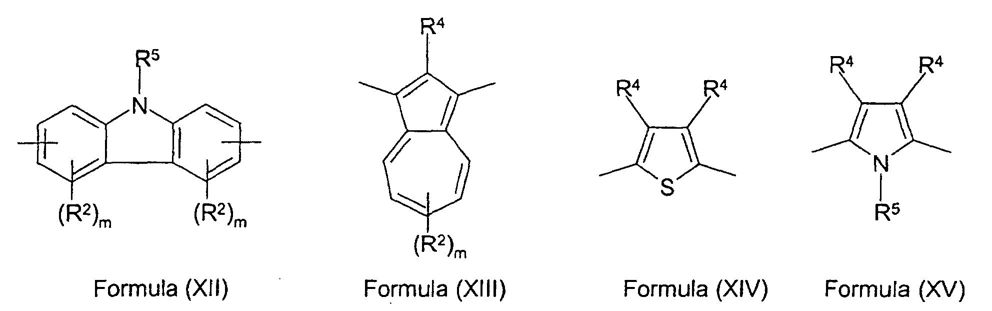 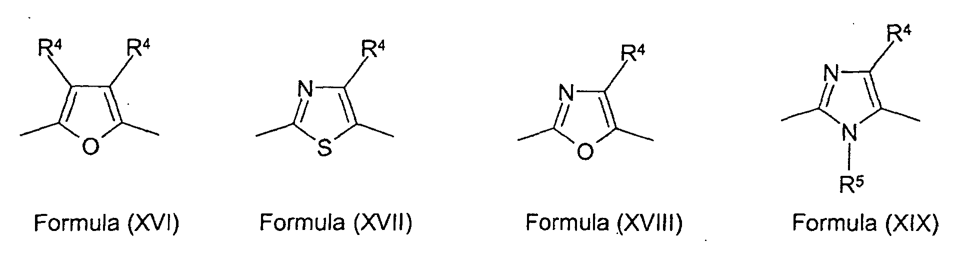 where the symbols R1 to R5 and the indices n and m are as defined in claim 1 and the further symbols have the following meanings:
Ar1, Ar2, Ar3 are identical or different on each occurrence and are aromatic or heteroaromatic hydrocarbons which have from 2 to 40 carbon atoms and may also be substituted by one or more nonaromatic radicals R1,
are present.

16. A polymer as claimed in one or more of claims 11 to 15, **characterized in that** at least one further structural element has electron transport properties.

17. A polymer as claimed in claim 16, **characterized in that** at least one of the following structural elements pyridine derivatives, pyrimidine derivatives, pyridazine derivatives, pyrazine derivatives, oxadiazole derivatives, quinoline derivatives, quinoxaline derivatives or phenazine derivatives is present.

18. A polymer as claimed in claim 16 and/or 17, **characterized in that** at least 1% and not more than 70% of one or more of the structural units of the formulae (XX) to (XXX), 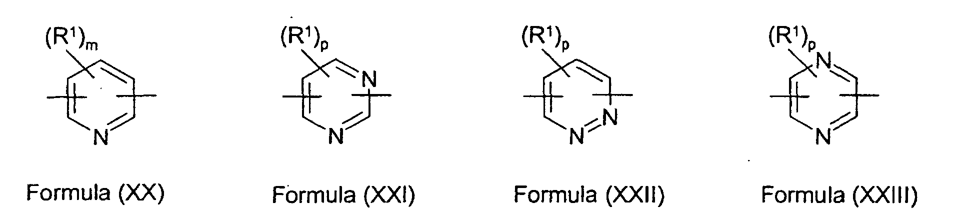 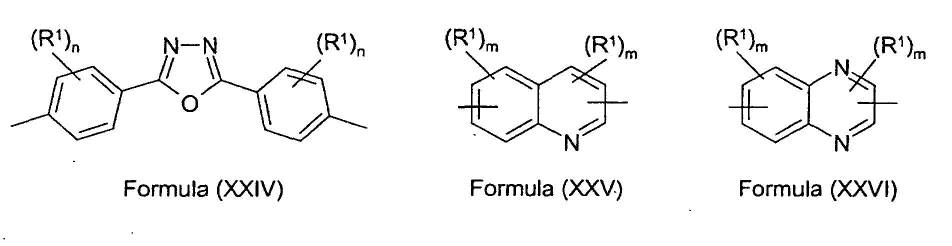 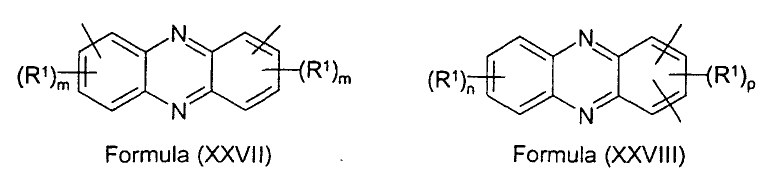 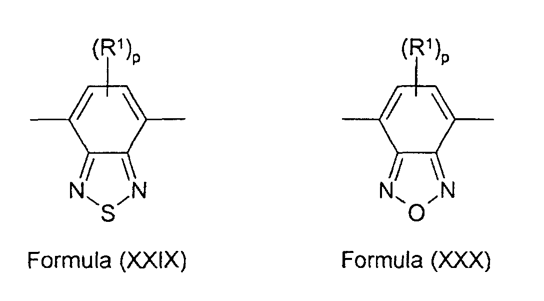 where the symbol R1 and the indices n and m are as defined in claim 1 and the index
p is 0, 1 or 2, preferably 0 or 1.
are present.

19. A polymer as claimed in claim 11, **characterized in that** it comprises at least one further structural element having electron transport properties and at least one further structural element having hole transport properties.

20. A polymer as claimed in claim 19, **characterized in that** it comprises one or more structural elements of the formulae (III) to (XIX) defined in claim 15 together with one or more structural elements of the formulae (XX) to (XXX) defined in claim 18.

21. A polymer as claimed in claim 19 and/or 20, **characterized in that** the polymer further comprises one or more structural units of the formulae (XXXI) to (XXXXV), 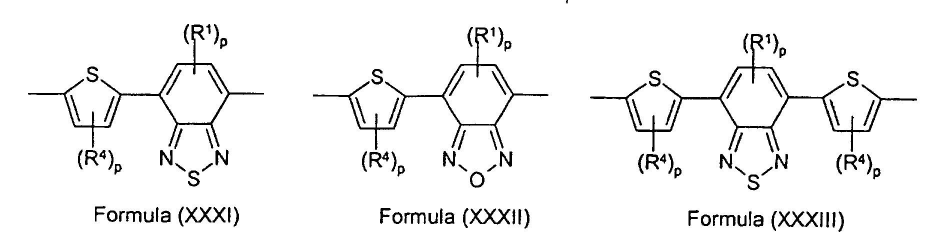 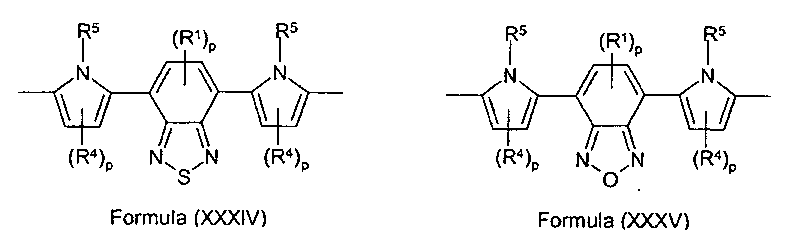 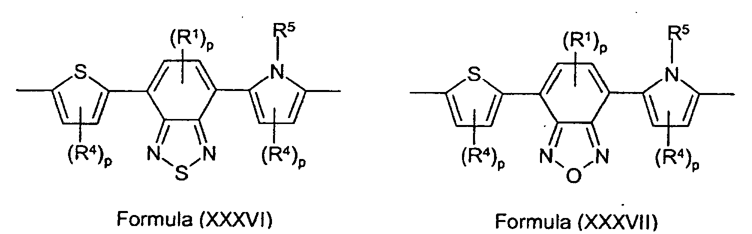 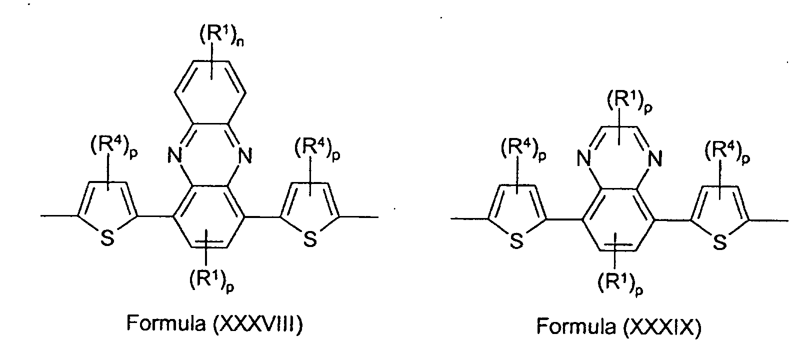 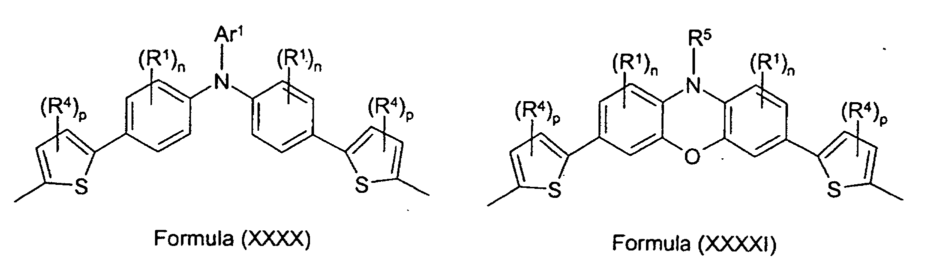 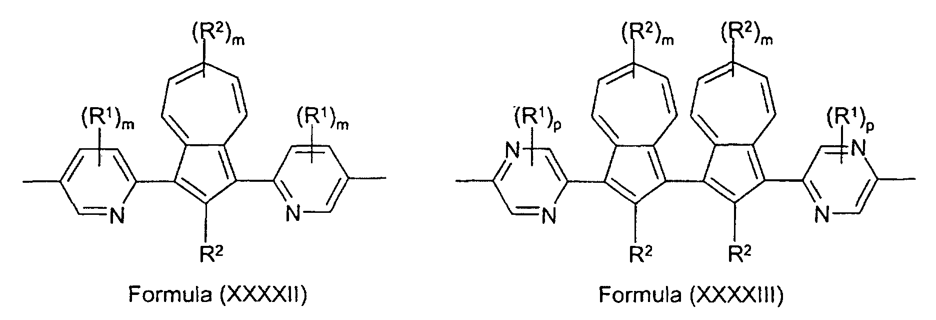 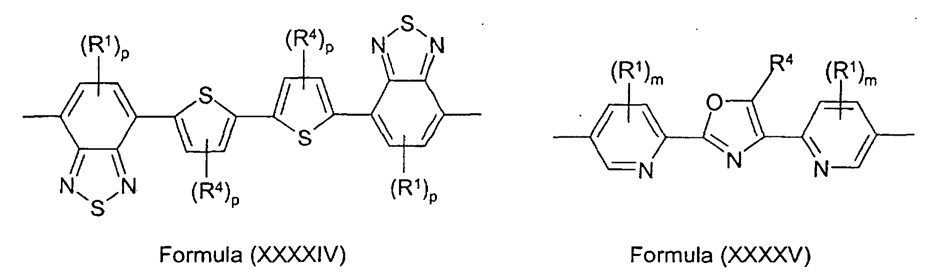 where the symbols and indices are defined in claims 1 and 18.

22. A polymer as claimed in one or more of claims 11 to 21 which further comprises at least one additional aromatic or other conjugated structure which has no influence or only little influence on the charge carrier mobilities.

23. A polymer as claimed in claim 22, **characterized in that** it comprises aromatic structures having from 6 to 40 carbon atoms or stilbene or bisstyrylarylene derivatives which may be substituted by one or more nonaromatic radicals R1.

24. A polymer as claimed in claim 22 and/or 23, **characterized in that** 1,4-phenylene, 1,4-naphthylene, 1,4- or 9,1 0-anthracenylene, 1,6- or 2,7- or 4,9-pyrene, 3,9- or 3,1 0-perylene, 2,7- or 3,6-phenanthrene, 4,4'-biphenylene, 4,4"-terphenylene, 4,4'-bi-1,1'-naphthylene, 4,4'-stilbene or 4,4"-bisstyrylarylene derivatives are incorporated.

25. A polymer as claimed in one or more of claims 11 to 24, **characterized in that** organometallic complexes are incorporated into the main chain.

26. A polymer as claimed in claim 25, **characterized in that** d transition metal complexes of the higher metals of the iron, cobalt and nickel triads, i.e. complexes of ruthenium, osmium, rhodium, iridium, palladium and platinum, are present.

27. A polymer as claimed in claim 26 and/or 27, **characterized in that** one or more of the structural units of the formulae (XXXXVI) to (XXXXIX), 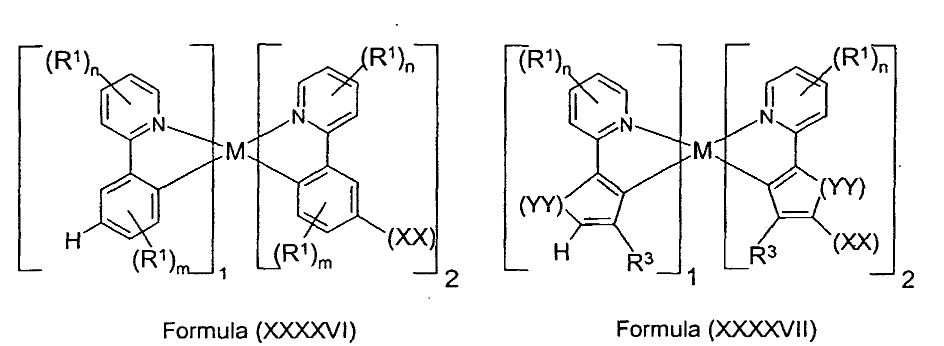 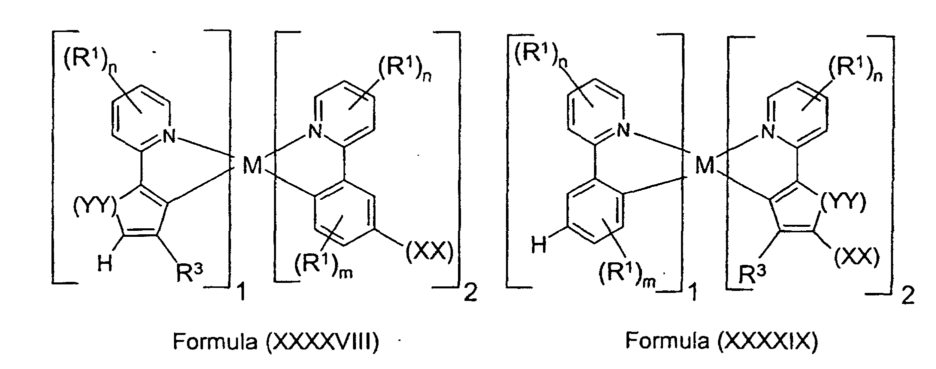 where the symbols R1 and R3 and the indices n and m are as defined in claim 1 and
M is Rh or lr,
XX is the point of linkage in the polymer, and
YY is identical or different on each occurrence and is O, S or Se,
are additionally present.

28. The use of one or more of the polymers as claimed in one or more of claims 1 to 27 in a PLED, in particular as electroluminescence material.

29. A PLED having one or more active layers, wherein at least one of these active layers comprises one or more polymers as claimed in one or more of claims 1 to 27.

30. An electronic component (device) comprising one or more polymers as claimed in one or more of claims 1 to 27.

31. An organic integrated circuit (O-lC), organic field effect transistor (OFET), organic thin film transistor (OTFT), organic solar cell (O-SC) or organic laser diode (O laser), **characterized in that** it comprises one or more polymers as claimed in one or more of claims 1 to 27.

## Revendications

1. Polymères conjugués contenant des unités récurrentes répondant à la formule (I) 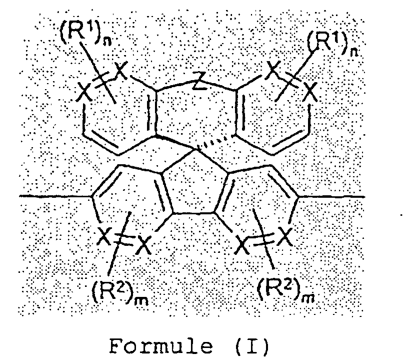 et des unités récurrentes répondant à la formule (II) 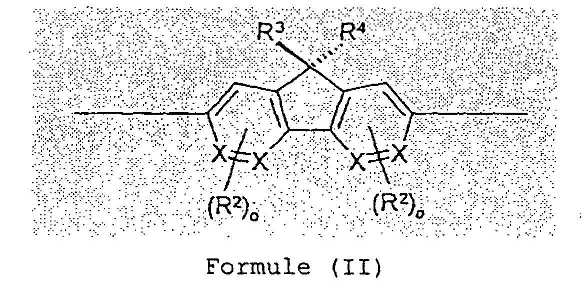 dans lesquels la part des unités répétitives du type de la formule (I) et de la formule (II) prises conjointement représente au moins 20 % et le rapport des unités répétitives du type de la formule (I) relativement au type de la formule (II) se situe dans la plage allant de 1 : 10 à 10 : 1, et les symboles et indices ont la signification suivante :
X représente dans chaque cas, de manière identique ou différente, C-H, CR¹ ou N ;
Z représente dans chaque cas, de manière identique ou différente, une liaison chimique simple, un groupe CR³R⁴, un groupe -CR³R⁴-CR³R⁴, un groupe -CR³=CR⁴, O, S, N-R⁵, C=O, C=CR³R⁴ ou SiR³R⁴;
R¹ représente dans chaque cas, de manière identique ou différente, une chaîne alkyle ou alcoxy linéaire, ramifiée ou cyclique ayant de 1 à 22 atomes de carbone, dans laquelle un ou plusieurs atomes de carbone non adjacents peuvent également être remplacés par N-R⁵, O, S, -CO-O-, O-CO-O, un ou plusieurs atomes d'hydrogène pouvant également être remplacés par le fluor, un groupe aryle ou aryloxy avec de 5 à 40 atomes de carbone, où un ou plusieurs atomes de carbone peuvent également être remplacés par O, S ou N, lesquels peuvent également être substitués par un ou plusieurs restes non aromatiques R¹, ou bien Cl, F, CN, N (R⁵) ₂, où deux ou plusieurs restes R¹ peuvent également former conjointement un système cyclique ;
R² représente dans chaque cas, de manière identique ou différente, une chaîne alkyle ou alcoxy linéaire, ramifiée ou cyclique ayant de 1 à 22 atomes de carbone, dans laquelle un ou plusieurs atomes de carbone non adjacents peuvent également être remplacés par N-R⁵, O, S, -CO-O-, O-CO-O, un ou plusieurs atomes d'hydrogène pouvant également être remplacés par le fluor, un groupe aryle ou aryloxy avec de 5 à 40 atomes de carbone, où un ou plusieurs atomes de carbone peuvent également être remplacés par O, S ou N, lesquels peuvent également être substitués par un ou plusieurs restes non aromatiques R¹, ou bien CN ;
R³, R⁴ représentent dans chaque cas, de manière identique ou différente, une chaîne alkyle linéaire, ramifiée ou cyclique ayant de 1 à 22 atomes de carbone, dans laquelle un ou plusieurs atomes de carbone non adjacents peuvent également être remplacés par N-R⁵, O, S, -CO-O-, O-CO-O, où un ou plusieurs atomes d'hydrogène peuvent également être remplacés par le fluor, un groupe aryle avec de 5 à 40 atomes de carbone, où un ou plusieurs atomes de carbone peuvent également être remplacés par O, S ou N, lesquels peuvent également être substitués par un ou plusieurs restes non aromatiques R¹, ou bien CN ; les deux restes R³ et R⁴ peuvent également former conjointement un cycle ne conduisant cependant pas aux structures répondant à la Formule (I) ;
R⁵ représente dans chaque cas, de manière identique ou différente, H, une chaîne alkyle linéaire, ramifiée ou cyclique ayant de 1 à 22 atomes de carbone, dans laquelle un ou plusieurs atomes de carbone non adjacents peuvent également être remplacés par O, S, -CO-O-, O-CO-O, un ou plusieurs atomes d'hydrogène pouvant également être remplacés par le fluor, un groupe aryle avec de 5 à 40 atomes de carbone, où un ou plusieurs atomes de carbone peuvent également être remplacés par O, S ou N, lesquels peuvent également être substitués par un ou plusieurs restes non aromatiques R¹ ;
n vaut, dans chaque cas, de manière identique ou différente, 0, 1, 2, 3 ou 4 ;
m vaut, dans chaque cas, de manière identique ou différente, 0, 1, 2 ou 3 ;
o vaut, dans chaque cas, de manière identique ou différente, 0, 1, 2 ou 3 ;
avec la précision que, dans au moins une unité répondant à la formule (I), au moins un indice n et/ou m est différent de 0.

2. Polymères selon la revendication 1, **caractérisés en ce que** le symbole X= représente C-H ou C-R¹.

3. Polymères selon la revendication 1 et/ou 2, **caractérisés en ce que** le symbole Z représente une liaison chimique simple

4. Polymères selon une ou plusieurs des revendications 1 à 3, **caractérisés en ce que** :
R¹ représente dans chaque cas, de manière identique ou différente, une chaîne alkyle ou alcoxy linéaire, ramifiée ou cyclique ayant.de 1 à 10 atomes de carbone, dans laquelle un ou plusieurs atomes d'hydrogène peuvent également être remplacés par le fluor, un groupe aryle avec de 6 à 14 atomes de carbone, qui est également substitué par un ou plusieurs restes non aromatiques R¹;

5. Polymères selon une ou plusieurs des revendications 1 à 4, **caractérisés en ce que** :
R¹ représente dans chaque cas, de manière identique ou différente, une chaîne alkyle ou alcoxy linéaire ou ramifiée ayant de 1 à 8 atomes de carbone, ou un groupe aryle avec de 6 à 10 atomes de carbone, qui est également substitué par un ou plusieurs restes non aromatiques R¹;
n vaut, de manière identique ou différente, 1 ou 2.

6. Polymères selon une ou plusieurs des revendications 1 à 5, **caractérisés en ce que** :
R² représente dans chaque cas, de manière identique ou différente, une chaîne alkyle ou alcoxy linéaire ou ramifiée ayant de 1 à 10 atomes de carbone, un ou plusieurs atomes d'hydrogène pouvant être également remplacés par le fluor, un groupe aryle ou aryloxy avec de 6 à 14 atomes de carbone, qui peuvent également être substitués par un ou plusieurs restes non aromatiques R¹, ou bien CN ;
o, m valent, dans chaque cas, de manière identique ou différente, 0 ou 1.

7. Polymères selon une ou plusieurs des revendications 1 à 6, **caractérisés en ce que** :
R² représente dans chaque cas, de manière identique ou différente, une chaîne alkyle ou alcoxy linéaire ou ramifiée ayant de 1 à 8 atomes de carbone, un ou plusieurs atomes d'hydrogène pouvant également être remplacés par le fluor, un groupe aryle avec de 6 à 10 atomes de carbone, qui peuvent également être substitués par un ou plusieurs restés non aromatiques R¹ ;
o, m valent, dans chaque cas, de manière identique ou différente, 0 ou 1, et pour au moins 50 % de toutes les unités répétitives présentes dans le polymère conforme à la formule (I) et (II) o et m valent 0.

8. Polymères selon une ou plusieurs des revendications 1 à 7, **caractérisés en ce que** :
R³, R⁴ représentent dans chaque cas, de manière identique ou différente, une chaîne alkyle linéaire, ramifiée ou cyclique ayant de 1 à 10 atomes de carbone, dans laquelle un ou plusieurs atomes de carbone non adjacents peuvent également être remplacés par O, où un ou plusieurs atomes d'hydrogène peuvent également être remplacés par le fluor, un groupe aryle avec de 5 à 40 atomes de carbone, où un ou plusieurs atomes de carbone peuvent être remplacés par O, S ou N, lesquels peuvent également être substitués par un ou plusieurs restes non aromatiques R¹.

9. Polymères selon une ou plusieurs des revendications 1 à 8, **caractérisés en ce que** :
R³, R⁴ représentent dans chaque cas, de manière identique ou différente, un groupe aryle ayant de 6 à 14 atomes de carbone qui peuvent également être substitués par un ou plusieurs restes non aromatiques R¹.

10. Polymères selon une ou plusieurs des revendications 1 à 9, **caractérisés en ce que** :
R³, R⁴ représentent dans chaque cas, de manière identique ou différente, un groupe aryle ayant de 6 à 14 atomes de carbone qui peuvent également être substitués par un ou plusieurs restes non aromatiques R¹ et dans lesquels, pour chaque unité répondant à la formule (II), les substituants R³ et R⁴ sont différents l'un de l'autre.

11. Polymères selon une ou plusieurs des revendications 1 à 10, **caractérisés en ce qu'**ils renferment d'autre unités répétitives présentant d'autres structures que celles répondant à la formule (I) ou (II).

12. Polymères selon la revendication 11, **caractérisés en ce qu'**au moins un autre élément structurel présente des propriétés de transport de charge.

13. Polymères selon la revendication 11 et/ou 12, **caractérisés en ce qu'**au moins un autre élément structurel présente des propriétés de transport de trous.

14. Polymères selon une ou plusieurs des revendications 11 à 13, **caractérisés en ce qu'**ils contiennent au moins un des éléments structurels suivants : dérivés de la triarylamine, dérivés de la benzidine, dérivés de la tétraarylène-paraphénylènediamine, dérivés de la phénothiazine, dérivés de la phénoxazine, dérivés de la dihydrophénazine, dérivés du thianthrène, dérivés de la benzo-p-dioxine, dérivés de la phénoxathiine, dérivés du carbazole, dérivés de l'azulène, dérivés du thiophène, dérivés du pyrrole ou dérivés du furane.

15. Polymères selon une ou plusieurs des revendications 11 à 14, **caractérisés en ce qu'**ils contiennent au moins 1 % et au plus 70 % d'une ou de plusieurs unités structurelles répondant aux formules (III) à (XIX), 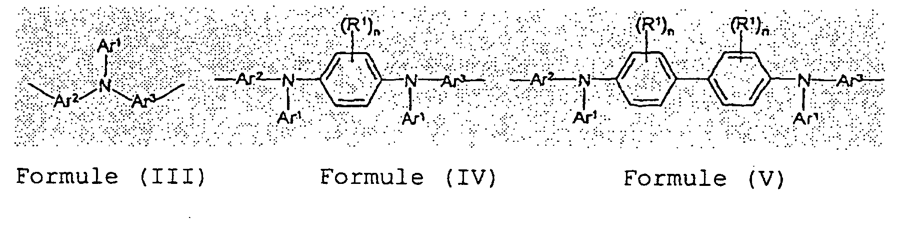 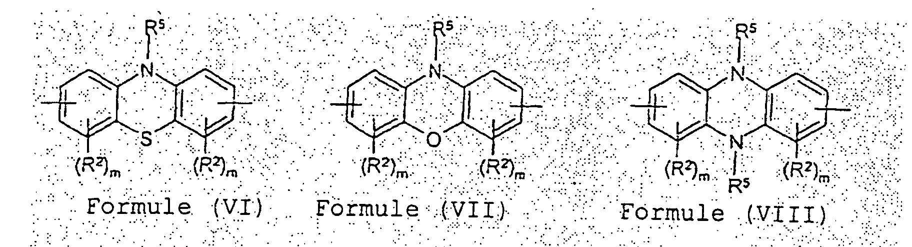 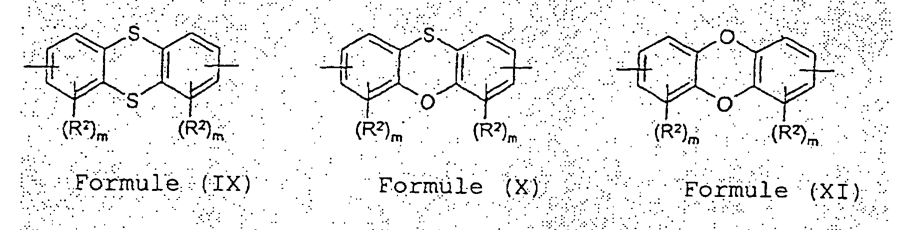 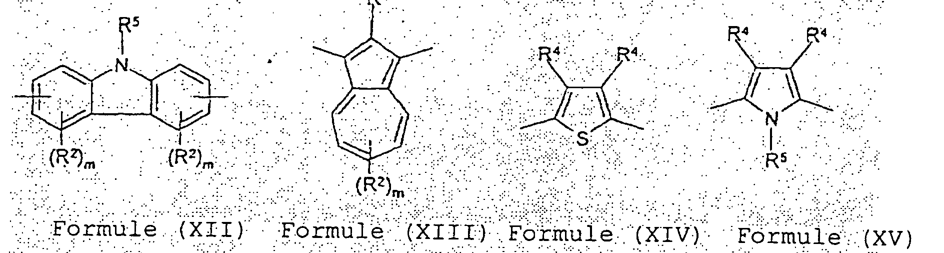 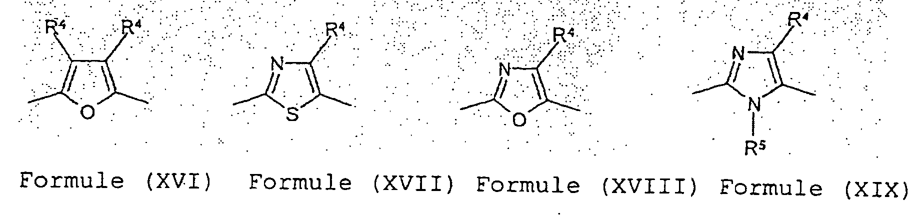 dans lesquelles les symboles R¹ à R⁵ et les indices n et m ont les significations indiquées dans la revendication 1, et les autres symboles
Ar¹, Ar², Ar³ représentent, dans chaque cas, de manière identique ou différente, des hydrocarbures aromatiques ou hétéroaromatiques ayant de 2 à 40 atomes de carbone pouvant être substitués par un ou plusieurs restes non aromatiques R¹.

16. Polymères selon une ou plusieurs des revendications 11 à 15, **caractérisés en ce qu'**au moins un autre élément structurel présente des propriétés de transport d'électrons.

17. Polymères selon la revendication 16, **caractérisés en ce qu'**ils contiennent au moins un des éléments structurels suivants : dérivés de la pyridine, dérivés de la pyrimidine, dérivés de la pyridaziné, dérivés de la pyrazine, dérivés de l'oxadiazole, dérivés de la quinoline, dérivés de la quinoxaline ou dérivés de la phénazine.

18. Polymères selon la revendication 16 et/ou 17, **caractérisés en ce qu'**ils contiennent au moins 1 % et au plus 70 % d'une ou de plusieurs unités structurelles répondant aux formules (XX) à (XXX), 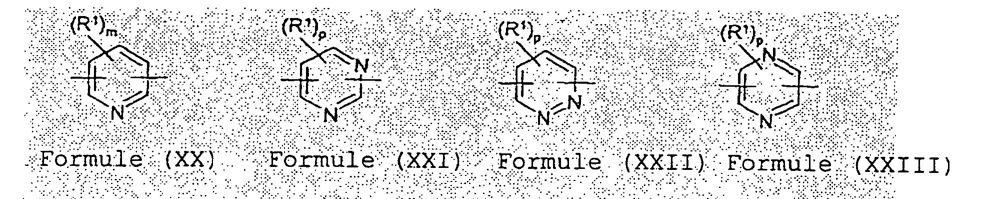 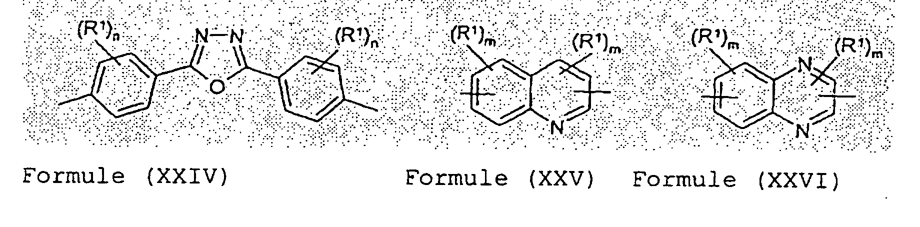 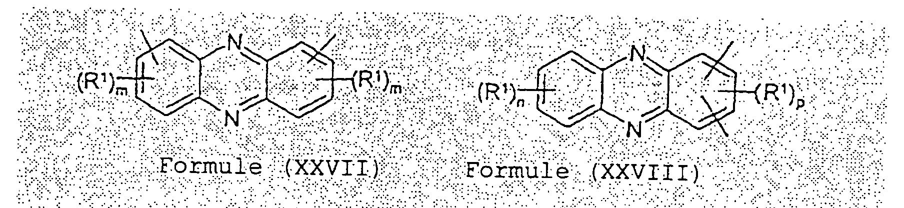 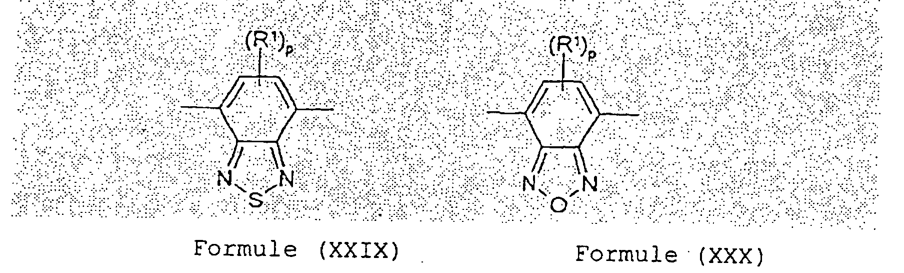 dans lesquelles le symbole R¹ et les indices n et m ont les significations indiquées dans la revendication 1, et l'indice
p vaut 0, 1 ou 2, de préférence 0 ou 1.

19. Polymères selon la revendication 11, **caractérisés en ce qu'**ils renferment au moins un autre élément structurel présentant des propriétés de transport d'électrons ainsi qu'au moins un autre élément structurel présentant des propriétés de transport de trous.

20. Polymères selon la revendication 19, **caractérisés en ce qu'**ils renferment aussi bien un ou plusieurs éléments structurels répondant aux formules (III) à (XIX) définies dans la revendication 15, qu'un ou plusieurs éléments structurels répondant aux formules (XX) à (XXX) définies dans la revendication 18.

21. Polymères selon la revendication 19 et/ou 20, **caractérisés en ce que** le polymère renferme en outre une ou plusieurs unités structurelles répondant aux formules (XXXI) à (XXXXV) 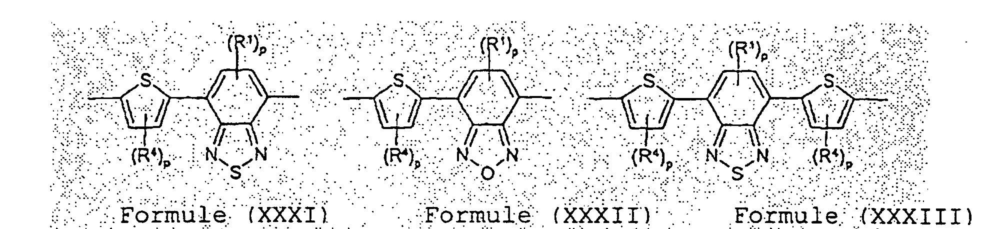 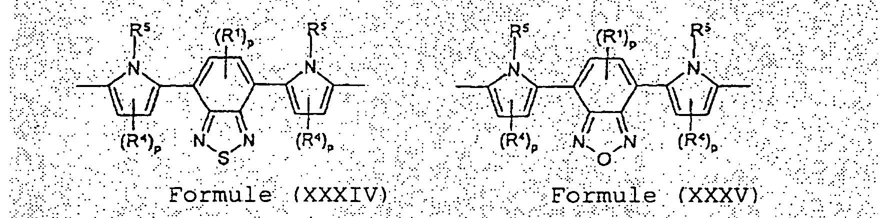 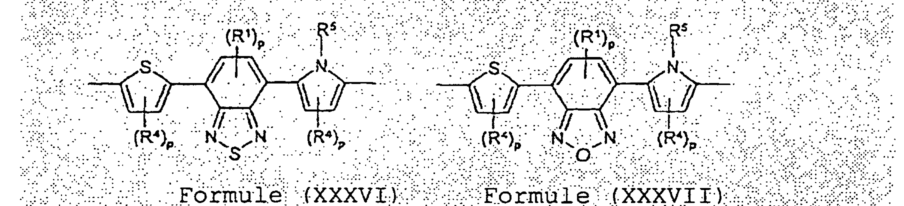 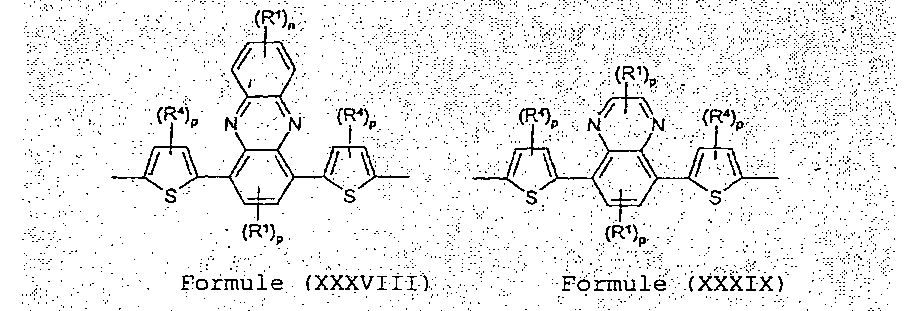 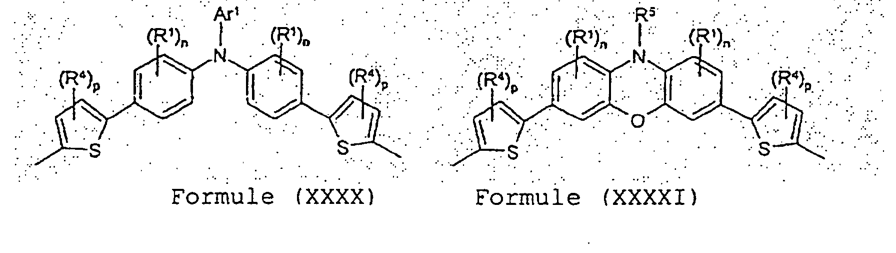 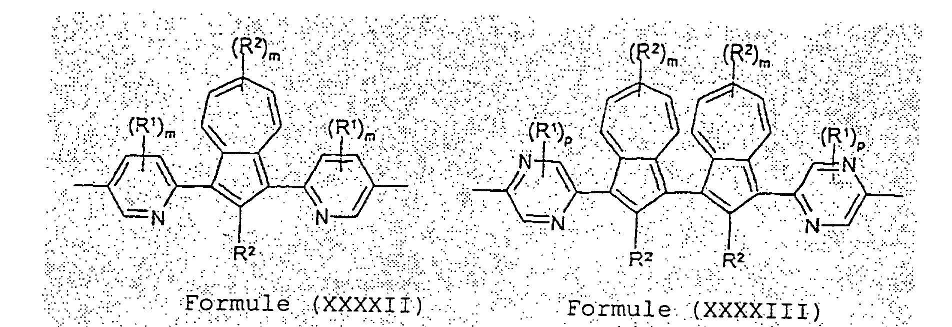 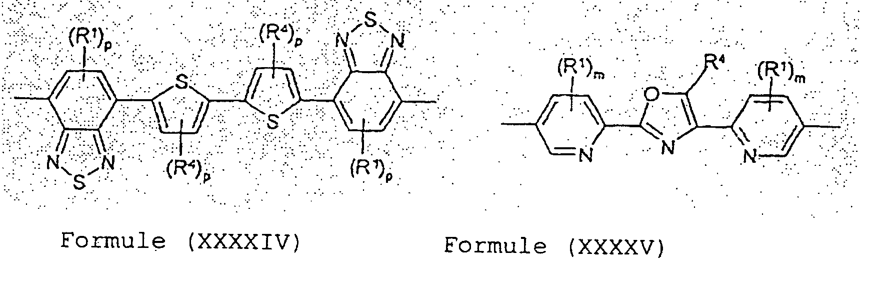 dans lesquelles les symboles et indices ont les significations indiquées dans les revendication 1 et 18:

22. Polymères selon une ou plusieurs des revendications 11 à 21, qui présentent au moins encore une autre structure aromatique ou une structure conjuguée différente n'interférant pas ou très peu avec les mobilités des porteurs de charge.

23. Polymères selon la revendication 22, **caractérisés en ce qu'**ils présentent des structures aromatiques ayant de 6 à 40 atomes de carbone ou des dérivés du stilbène ou de bis-styrylarylène pouvant être substitués par un ou plusieurs restes non aromatiques R¹.

24. Polymères selon la revendication 22 et/ou 23, **caractérisés en ce que** sont incorporés des dérivés du 1,4-phénylène, des dérivés du 1,4-naphthylène, des dérivés du 1,4-anthracénylène ou du 9,10-anthracénylène, des dérivés du 1,6-pyrène, du 2,7-pyrène ou du 4,9-pyrène, des dérivés du 3,9-pérylène ou du 3,10-pérylène, des dérivés du 2,7-phénanthrène ou du 3,6-phénanthrène, des dérivés du 4,4'-biphénylène, des dérivés du 4,4''-terphénylène, des dérivés du 4,4' -bi-1,1' naphtylène, des dérivés du 4,4'-stilbène ou des dérivés du 4,4''-bisstyrylarylène.

25. Polymères selon une ou plusieurs des revendications 11 à 24, **caractérisés en ce que** des complexes métallo-organiques sont incorporés dans la chaîne principale.

26. Polymères selon la revendication 25, **caractérisés en ce qu'**ils contiennent des complexes de métaux de transition des métaux supérieurs des triades du fer, du cobalt et du nickel, c'est-à-dire des complexes de ruthénium, d'osmium, de rhodium, d'iridium, de palladium et de platine.

27. Polymères selon la revendication 26 et/ou 27, **caractérisés en ce qu'**ils renferment en outre une ou plusieurs unités structurelles répondant aux formules (XXXXVI) à (XXXXIX), 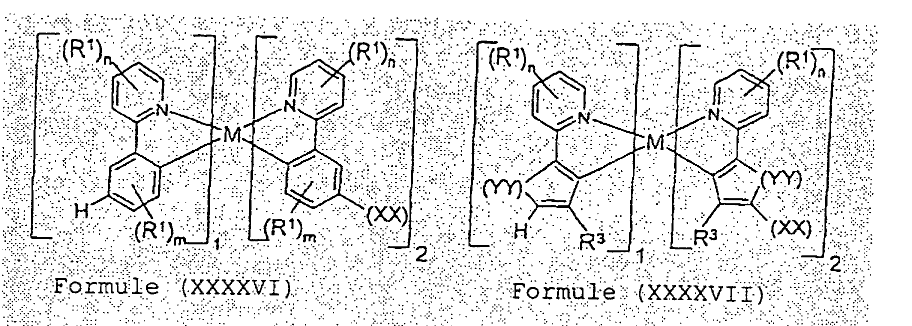 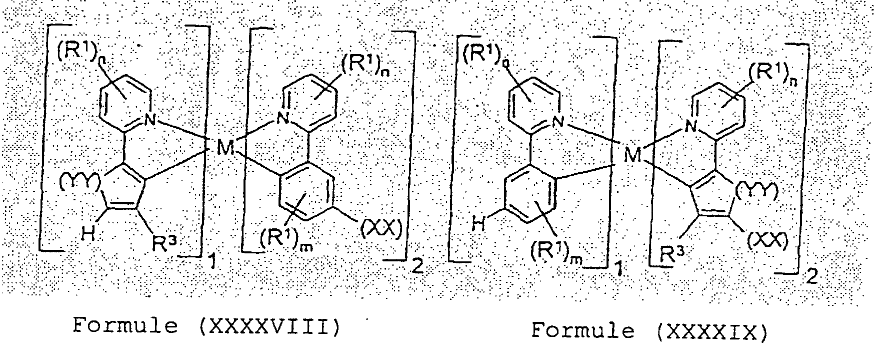 dans lesquelles les symboles R¹ et R³ et les indices n et m ont les significations indiquées dans la revendication 1, et
M représente un atome de Rh ou Ir,
XX représente l'emplacement d'une liaison au sein du polymère et
YY représente dans chaque cas, de manière identique ou différente, un atome de O, S ou Se.

28. Utilisation d'un ou de plusieurs des polymères selon une ou plusieurs des revendications 1 à 27 dans une diode électroluminescente polymérique (PLED), notamment à titre de matériau électroluminescent.

29. Diode électroluminescente polymérique comportant une ou plusieurs couches actives, dans laquelle au moins une de ces couches actives renferme un ou plusieurs polymères conformes à l'invention selon une ou plusieurs des revendications 1 à 27.

30. Composant électronique (device) contenant un ou plusieurs polymères conformes à l'invention selon une ou plusieurs des revendications 1 à 27.

31. Circuits intégrés organiques (O-IC), transistors organiques à effet de champ (OFET), transistors organiques à couches minces (OTFT), cellules solaires organiques (O-SC) ou diodes laser organiques (O-laser), **caractérisés en ce qu'**ils contiennent un ou plusieurs polymères selon une ou plusieurs des revendications 1 à 27.
